(19)

**Europäisches Patentamt**
**European Patent Office**
**Office européen des brevets**

(11)  **EP 4 703 387 A1**

(12)  **EUROPEAN PATENT APPLICATION**
published in accordance with Art. 153(4) EPC

(43) Date of publication:
04.03.2026  Bulletin 2026/10

(21) Application number: 24796673.2

(22) Date of filing: 26.03.2024

(51) International Patent Classification (IPC):
C07K 16/28 (2006.01)    A61K 39/395 (2006.01)
A61P 1/04 (2006.01)    A61P 9/10 (2006.01)
A61P 29/00 (2006.01)    A61P 31/00 (2006.01)
A61P 37/02 (2006.01)    A61P 37/08 (2006.01)
A61P 43/00 (2006.01)    C12N 1/15 (2006.01)
C12N 1/19 (2006.01)    C12N 1/21 (2006.01)
C12N 5/10 (2006.01)    C12N 15/13 (2006.01)
C12N 15/63 (2006.01)    C12P 21/08 (2006.01)
G01N 33/53 (2006.01)    G01N 33/531 (2006.01)

(52) Cooperative Patent Classification (CPC):
A61K 39/395; A61P 1/04; A61P 9/10; A61P 29/00;
A61P 31/00; A61P 37/02; A61P 37/08; A61P 43/00;
C07K 16/00; C07K 16/28; C12N 5/10; C12N 15/63;
C12N 15/74; C12N 15/80; C12N 15/81;    (Cont.)

(86) International application number:
PCT/JP2024/012013

(87) International publication number:
WO 2024/224915 (31.10.2024 Gazette 2024/44)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 28.04.2023  JP 2023074961
07.07.2023  JP 2023112253

(71) Applicant: University of Tsukuba
Ibaraki 305-8577 (JP)

(72) Inventors:
• SHIBUYA Akira
Tsukuba-shi, Ibaraki 305-8577 (JP)

• ODA Chigusa
Tsukuba-shi, Ibaraki 305-8577 (JP)
• OKUWAKI Shun
Tsukuba-shi, Ibaraki 305-8577 (JP)
• TAKAHASHI Hiroshi
Tsukuba-shi, Ibaraki 305-8577 (JP)
• KODA Masao
Tsukuba-shi, Ibaraki 305-8577 (JP)

(74) Representative: Dehns
10 Old Bailey
London EC4M 7NG (GB)

Remarks:
The complete document including Reference
Table(s) and the Sequence Listing(s) can be
downloaded from the EPO website

(54) **ANTI-HUMAN CD300A MONOCLONAL ANTIBODY AND ANTIGEN-BINDING FRAGMENT THEREOF**

(57)    Provided is an antibody that binds to at least one of human CD300A-R and human CD300A-Q, does not bind to human CD300C, and inhibits the binding between human CD300A and a ligand thereof. An anti-human CD300A monoclonal antibody or an antigen-binding fragment thereof satisfies a requirement (i), (ii), or (iii) as described in the specification.

EP 4 703 387 A1

(52)  Cooperative Patent Classification (CPC): (Cont.)
      **G01N 33/53; G01N 33/531**

**Description**

Technical Field

The present invention relates to an anti-human CD300A monoclonal antibody and an antigen-binding fragment thereof.

Background Art

The CD300 family is a type I transmembrane glycoprotein having one immunoglobulin-like domain located extracellularly. The CD300 family is mainly expressed in myeloid cells, and it has been reported that seven proteins having similar extracellular regions form the CD300 family in humans by analysis using a database.

Within the human CD300 family, human CD300A (hereinafter, also simply referred to as CD300A) and human CD300F have an immunoreceptor tyrosine-based inhibitory motif (ITIM) in their intracellular regions, and function as inhibitory CD300 that transmits an inhibitory signal (Non Patent Literature 1). On the other hand, some members of the human CD300 family, including human CD300B and human CD300C (hereinafter also simply referred to as CD300C), are activating-type CD300 that transmits activation signals, for example, by associating with adaptor molecules containing an immunoreceptor tyrosine-based activation motif (ITAM) (for example, FcRγ or DAP12).

When CD300A is activated, tyrosine in the ITIM is phosphorylated and further recruits phosphatases such as SHP1, SHP2, and SHIP, and these phosphatases subsequently transmit intracellular inhibitory signals in later steps (Non Patent Literature 2). In humans, CD300A is expressed in several types of immune cells, such as myeloid cells, NK cells, T cells, or subsets of B cells (Non Patent Literature 1). Phosphatidylserine (PS) and phosphatidylethanolamine (PE) have been identified as natural ligands of CD300A, and it is known that PS and PE are exposed on the surface of apoptotic cells and dead cells (Non Patent Literature 3).

It has been reported in numerous studies that CD300A plays an important role in the regulation of immune responses (Non Patent Literatures 4 to 9). In addition, it has been shown in mice that inhibiting the binding between CD300a and a ligand thereof is effective for the treatment of diseases, and thus it is considered to be effective also in humans.

For example, in mice, binding of PS to CD300a on, for example, mast cells enhances the inhibitory signaling of CD300a and suppresses activation of such cells. Accordingly, it has been reported that inhibition of the binding between PS and CD300a using an anti-CD300a antibody maintains mast cell activation and enables treatment of allergic diseases (Patent Literature 1).

In addition, it is also known that CD300a is activated through binding to PS and suppresses CD300b-dependent phagocytosis in macrophages. Therefore, it has been reported that inhibition of the binding between PS and CD300a using an anti-CD300a antibody suppresses the activity of CD300a, thereby promoting CD300b-dependent phagocytosis, suppressing the accumulation of dead cells in tissues at the ischemic site, and enabling the treatment of ischemic diseases (Patent Literature 2).

Since CD300A and CD300C have a significantly high amino acid sequence identity of about 90% in the extracellular domains, it is difficult to obtain a specific monoclonal antibody against CD300A (Non Patent Literature 10). Therefore, in order to examine the expression and function of CD300A, a method has been employed in which an anti-CD300A/C double antibody that recognizes both CD300A and CD300C and an anti-CD300C antibody that recognizes only CD300C are used in combination, and the result obtained using the anti-CD300C antibody is subtracted from the result obtained using the anti-CD300A/C double antibody to exclude the expression of CD300C (Non Patent Literatures 5 and 8). However, this method has limitations in that, when both CD300A and CD300C are expressed on the cell surface, it is difficult to examine the direct function of CD300A in certain cell populations, such as monocytes and dendritic cells (Non Patent Literatures 5 and 9).

A monoclonal antibody against human CD300A is reported in, for example, Patent Literature 1. In addition, Non Patent Literature 10 reports a specific anti-CD300A monoclonal antibody capable of distinguishing between CD300A and CD300C.

In the Ig domain (rs2272111) of human CD300A, there is a non-synonymous single nucleotide polymorphism (SNP) in which the arginine at position 111 is substituted with glutamine (Arg111Gln, R111Q). For example, it has been reported that about 80% of Japanese individuals have arginine-type CD300A (CD300A-R), while about 20% of Japanese individuals have glutamine-type CD300A (CD300A-Q) (Non Patent Literature 11). In addition, genetic linkage studies have suggested that Arg111Gln may be associated with susceptibility to psoriasis (Non Patent Literature 12). Furthermore, the structure of CD300A elucidated by Simhadri et al. reveals that Arg111 is located near the ligand-binding domain, and the point mutation (Arg111Gln) significantly reduces the binding efficiency of CD300A to dead cells (Non Patent Literature 3).

Citation List

Patent Literature

Patent Literature 1: WO 2014/073529 A
Patent Literature 2: WO 2019/107445 A

Non Patent Literature

Non Patent Literature 1: Borrego, F. The CD300 molecules: an emerging family of regulators of the immunesystem. Blood 121, 1951-1960 (2013).
Non Patent Literature 2: Y. Okoshi, et al. Requirement of the tyrosines at residues 258 and 270 of MAIR-I in inhibitory effect on degranulation from basophilic leukemia RBL-2H3. Int. Immnol. 17, 65-72 (2005).
Non Patent Literature 3: Simhadri, V.R. et al. Human CD300A binds to phosphatidylethanolamine and phosphatidylserine, and modulates the phagocytosis of dead cells. Blood 119, 2799-2809 (2012).
Non Patent Literature 4: Vitallae, J. et al. CD300A inhibits CD16-mediated NK cell effector functions in HIV-1-infected patients. Cell. Mol. Immnol. 16, 940-942 (2019).
Non Patent Literature 5: Zenarruzabeitia, O. et al. The expression and function of human CD300 receptors on blood circulating mononuclear cells are distinct in neonates and adults. Sci. Rep. 6, 32693 (2016).
Non Patent Literature 6: Bachelet, I. et al. The inhibitory receptor IRp60 (CD300A) is expressed and functional on human mast cells. J. Immunol. 175, 7989-7995 (2005).
Non Patent Literature 7: Yotsumoto, K. et al. Paired activating and inhibitory immunoglobulin-like receptors, MAIR-I and MAIR-II, regulate mast cell and macrophage activation. J. Exp. Med. 198, 223-233 (2003).
Non Patent Literature 8: Vitallae, J. et al. Altered expression of CD300A inhibitory receptor on CD4+ T cells from human immunodeficiency virus-1-infected patients: association with disease progression markers. Front. Immunol. 9, 1709 (2018).
Non Patent Literature 9: Simhadri, V.R. et al. CD300C is an activating receptor expressed on human monocytes. J. Innate Immun. 5, 389-400 (2013).
Non Patent Literature 10: Takahashi M. et al. Human CD300C delivers an Fc receptor-γ-dependent activating signal in mast cells and monocytes and differs from CD300A in ligand recognition. J. Biol. Chem. 288, 7662-75 (2013).
Non Patent Literature 11: Reference SNP (rs) Report rs2272111, dbSNP Short Genetic Variations, National Library of Medicine, [online], National Center for Biotechnology Information, [Searched on April 25, 2023], Internet <https://www.ncbi.nlm.nih.gov/snp/rs2272111>
Non Patent Literature 12: Speckman, R.A. et al. Novel immunoglobulin superfamily gene cluster, mapping to a region of hman chromosome 17q25, linked to psoriasis ssceptibility. Hum. Genet. 112, 31-41 (2003).

Summary of Invention

Technical Problem

Although it has been shown that the anti-CD300A monoclonal antibody TX49 reported in Patent Literature 1 binds to arginine-type (CD300A-R), it remains unclear whether it binds to CD300C or the glutamine-type (CD300A-Q). In addition, it was also unclear whether TX49 could inhibit the binding between CD300A and a ligand thereof.

Although a specific anti-CD300A monoclonal antibody (hereinafter, referred to as "Antibody A") capable of distinguishing between CD300A and CD300C, as reported in Non Patent Literature 10, has been shown to bind to the arginine-type (CD300A-R), it remains unclear whether it binds to the glutamine-type (CD300A-Q). In addition, it was also unclear whether Antibody A could inhibit the binding between CD300A and a ligand thereof.

The present inventors considered that, when an anti-CD300A antibody binds to at least one of human CD300A-R and human CD300A-Q, does not bind to human CD300C, and inhibits the binding between human CD300A and a ligand thereof, the function of CD300A can be directly examined even when both CD300A and CD300C are expressed on the cell surface, side effects caused by the anti-CD300A antibody binding to CD300C when administered to a human can be avoided, and further, it is useful for treatment of a disease caused by the function of human CD300A.

Therefore, an object of the present invention is to provide an anti-CD300A monoclonal antibody that binds to at least one of human CD300A-R and human CD300A-Q, does not bind to human CD300C, and inhibits the binding between human CD300A and a ligand thereof.

Furthermore, the present inventors considered that, when an anti-CD300A antibody binds to both arginine-type CD300A (CD300A-R) and glutamine-type CD300A (CD300A-Q), it is useful when the anti-CD300A antibody is used as a pharmaceutical, since both a human having arginine-type CD300A and a human having glutamine-type CD300A can be included as subjects for administration.

Therefore, one of the further objects of the present invention is to provide an anti-CD300A monoclonal antibody that binds to both human CD300A-R and human CD300A-Q, does not bind to human CD300C, and inhibits the binding between human CD300A and a ligand thereof.

In addition, the present inventors considered that, when the anti-CD300A antibody binds to one type of arginine-type CD300A (CD300A-R) and glutamine-type CD300A (CD300A-Q) and does not bind to the other type of CD300A, it is possible to discriminate whether CD300A is of an arginine-type or a glutamine-type, which is useful.

Therefore, one of the further objects of the present invention is to provide an anti-CD300A monoclonal antibody that binds to either human CD300A-R or human CD300A-Q, does not bind to the other, and does not bind to human CD300C.

Solution to Problem

The present inventors conducted extensive studies to solve the above problems. As a result, the present inventors have found that the above problems can be solved by having the following configuration, thereby completing the present invention.

The present invention relates to, for example, the following [1] to [18].

[1] An anti-human CD300A monoclonal antibody or an antigen-binding fragment thereof that satisfies the following requirement (i), (ii), or (iii):

(i) the anti-human CD300A monoclonal antibody or the antigen-binding fragment thereof including CDR1, CDR2 and CDR3 sequences of a heavy chain variable region having the amino acid sequence of SEQ ID NO: 7, and CDR1, CDR2 and CDR3 sequences of a light chain variable region having the amino acid sequence of SEQ ID NO: 8;

(ii) the anti-human CD300A monoclonal antibody or the antigen-binding fragment thereof including CDR1, CDR2 and CDR3 sequences of a heavy chain variable region having the amino acid sequence of SEQ ID NO: 15, and CDR1, CDR2 and CDR3 sequences of a light chain variable region having the amino acid sequence of SEQ ID NO: 16; or

(iii) the anti-human CD300A monoclonal antibody or the antigen-binding fragment thereof including CDR1, CDR2 and CDR3 sequences of a heavy chain variable region having the amino acid sequence of SEQ ID NO: 23, and CDR1, CDR2 and CDR3 sequences of a light chain variable region having the amino acid sequence of SEQ ID NO: 24.

[2] The anti-human CD300A monoclonal antibody or the antigen-binding fragment thereof according to [1], in which

the requirement (i) is satisfied,
the CDR1, CDR2, and CDR3 sequences of the heavy chain variable region are the amino acid sequences of SEQ ID NOs: 1, 2, and 3, respectively, and
the CDR1, CDR2, and CDR3 sequences of the light chain variable region are the amino acid sequences of SEQ ID NOs: 4, 5, and 6, respectively.

[3] The anti-human CD300A monoclonal antibody or the antigen-binding fragment thereof according to [1] or [2], in which the anti-human CD300A monoclonal antibody or the antigen-binding fragment thereof is a humanized antibody.

[4] The anti-human CD300A monoclonal antibody or the antigen-binding fragment thereof according to [2] or [3], in which
the anti-human CD300A monoclonal antibody or the antigen-binding fragment thereof includes:

a heavy chain variable region having an amino acid sequence that is at least 95% identical to the amino acid sequence of SEQ ID NO: 31; and
a light chain variable region having an amino acid sequence that is at least 95% identical to the amino acid sequence of SEQ ID NO: 32.

[5] The anti-human CD300A monoclonal antibody or the antigen-binding fragment thereof according to any one of [2] to [4], in which
the anti-human CD300A monoclonal antibody or the antigen-binding fragment thereof includes:

> a heavy chain variable region having the amino acid sequence of SEQ ID NO: 31; and
> a light chain variable region having the amino acid sequence of SEQ ID NO: 32.

[6] The anti-human CD300A monoclonal antibody or the antigen-binding fragment thereof according to [1], in which

> the requirement (ii) is satisfied,
> the CDR1, CDR2, and CDR3 sequences of the heavy chain variable region are the amino acid sequences of SEQ ID NOs: 9, 10, and 11, respectively, and
> the CDR1, CDR2, and CDR3 sequences of the light chain variable region are the amino acid sequences of SEQ ID NOs: 12, 13, and 14, respectively.

[7] The anti-human CD300A monoclonal antibody or the antigen-binding fragment thereof according to [1] or [6], in which
the anti-human CD300A monoclonal antibody or the antigen-binding fragment thereof includes:

> a heavy chain variable region having an amino acid sequence that is at least 80% identical to the amino acid sequence of SEQ ID NO: 15; and
> a light chain variable region having an amino acid sequence that is at least 80% identical to the amino acid sequence of SEQ ID NO: 16.

[8] The anti-human CD300A monoclonal antibody or the antigen-binding fragment thereof according to [1], in which

> the requirement (iii) is satisfied,
> the CDR1, CDR2, and CDR3 sequences of the heavy chain variable region are the amino acid sequences of SEQ ID NOs: 17, 18, and 19, respectively, and
> the CDR1, CDR2, and CDR3 sequences of the light chain variable region are the amino acid sequences of SEQ ID NOs: 20, 21, and 22, respectively.

[9] The anti-human CD300A monoclonal antibody or the antigen-binding fragment thereof according to [1] or [8], in which
the anti-human CD300A monoclonal antibody or the antigen-binding fragment thereof includes:

> a heavy chain variable region having an amino acid sequence that is at least 80% identical to the amino acid sequence of SEQ ID NO: 23; and
> a light chain variable region having an amino acid sequence that is at least 80% identical to the amino acid sequence of SEQ ID NO: 24.

[10] A nucleic acid encoding the anti-human CD300A monoclonal antibody or the antigen-binding fragment thereof according to any one of [1] to [9].

[11] A vector containing the nucleic acid according to [10].

[12] A transformant containing the vector according to [11].

[13] A pharmaceutical composition for treating or preventing a disease caused by a function of human CD300A, the pharmaceutical composition containing the anti-human CD300A monoclonal antibody or the antigen-binding fragment thereof according to any one of [3] to [5].

[14] The pharmaceutical composition according to [13], in which the disease is at least one selected from the group consisting of ischemic diseases, inflammatory diseases, infectious diseases, allergic diseases, organ fibrosis, and autoimmune diseases.

[15] The pharmaceutical composition according to [13], in which the disease is spinal cord injury.

[16] A pharmaceutical composition for treating or preventing a disease associated with a function of human CD300A, the pharmaceutical composition containing the anti-human CD300A monoclonal antibody or the antigen-binding fragment thereof according to any one of [3] to [5].

[17] The pharmaceutical composition according to [16], in which the disease is at least one selected from the group consisting of ischemic diseases, inflammatory diseases, infectious diseases, allergic diseases, organ fibrosis, autoimmune diseases, and spinal cord injury.

[18] A diagnostic agent containing the anti-human CD300A monoclonal antibody or the antigen-binding fragment thereof according to any one of [6] to [9].

Advantageous Effects of Invention

According to one aspect of the present invention, it is possible to provide an anti-CD300A monoclonal antibody that binds to at least one of human CD300A-R and human CD300A-Q, does not bind to human CD300C, and inhibits the binding between human CD300A and a ligand thereof.

According to one aspect of the present invention, it is possible to provide an anti-CD300A monoclonal antibody that binds to human CD300A-R and human CD300A-Q, does not bind to human CD300C, and inhibits the binding between human CD300A and a ligand thereof.

According to one aspect of the present invention, it is possible to provide an anti-CD300A monoclonal antibody that binds to either human CD300A-R or human CD300A-Q, does not bind to the other, and does not bind to human CD300C.

Brief Description of Drawings

Fig. 1 is a diagram comparing the amino acid sequences near the ligand-binding sites of human CD300A-R (huCD300A-R), human CD300A-Q (huCD300A-Q), and human CD300C (huCD300C).

Fig. 2 shows the binding properties of TX49, Antibody NO. 1, Antibody NO. 2, and Antibody NO. 3 to human CD300A-R, human CD300A-Q, and human CD300C.

Fig. 3 shows a function of TX49, Antibody NO. 1, Antibody NO. 2, and Antibody NO. 3 in inhibiting (neutralizing) the binding between human CD300A-R or human CD300A-Q and a ligand.

Fig. 4 is a graph obtained by quantifying the results of Fig. 3.

Fig. 5 shows a function of Antibody A in inhibiting (neutralizing) the binding between human CD300A-R or human CD300A-Q and a ligand.

Fig. 6 shows the results of analysis of expression of CD300A in human peripheral blood cells.

Fig. 7 is a view comparing the amino acid sequence near the ligand-binding site of human CD300A-R (huCD300A-R) with the amino acid sequence of the corresponding site of CD300A of Macaca fascicularis (M. f. CD300A).

Fig. 8 shows the binding properties of Antibody NO. 1 and Antibody NO. 2 to CD300A of Macaca fascicularis.

Fig. 9 shows the amino acid sequences of the variable regions and CDR1 to CDR3 of the heavy chains and light chains of Antibody NO. 1, Antibody NO. 2, and Antibody NO. 3. The underlined sequences represent the CDRs, which are CDR1, CDR2, and CDR3 in order from the N-terminus.

Fig. 10 is a diagram comparing the amino acid sequences of Antibody NO. 1 VH (SEQ ID NO: 7), Hu NO. 1 VH1, Hu NO. 1 VH2, Hu NO. 1 VH3 (SEQ ID NO: 31), and U96282 VH.

Fig. 11 is a diagram comparing the amino acid sequences of Antibody NO. 1 VL (SEQ ID NO: 8), Hu NO. 1 VL1, Hu NO. 1 VL2, Hu NO. 1 VL3 (SEQ ID NO: 32), and U96396 VL.

Fig. 12 shows a cDNA sequence encoding a heavy chain in pHu NO. 1U and an amino acid sequence estimated therefrom. The variable regions are shown in bold, the signal peptides are indicated in white text on a black background, and the CDRs are underlined. The CDRs are CDR1, CDR2, and CDR3 in order from the N-terminus.

Fig. 13 shows a cDNA sequence encoding a light chain in pHu NO. 1U and an amino acid sequence estimated therefrom. The variable regions are shown in bold, the signal peptides are indicated in white text on a black background, and the CDRs are underlined. The CDRs are CDR1, CDR2, and CDR3 in order from the N-terminus.

Fig. 14 shows the binding properties of Hu NO. 1U to human CD300A-R, human CD300A-Q, and human CD300C.

Fig. 15 is a diagram comparing the binding properties of Hu NO. 1U and Antibody NO. 1 to human CD300A-R and human CD300A-Q.

Fig. 16 shows a function of Hu NO. 1U and Antibody NO. 1 in inhibiting (neutralizing) the binding between human CD300A-R or human CD300A-Q and a ligand.

Fig. 17 is a graph showing the effect of Hu NO. 1U on enhancement of efferocytosis by human peripheral blood monocytes.

Fig. 18 is a graph showing changes in BMS score in a mouse spinal cord contusion injury model.

Fig. 19 is a graph showing changes in BMS subscore in a mouse spinal cord contusion injury model.

Fig. 20 is a photograph showing the result of HE staining of the spinal cord in a mouse spinal cord contusion injury model.

Fig. 21 is a graph showing the area of spinal cord injury in a mouse spinal cord contusion injury model.

Fig. 22 is a photograph showing the result of LFB staining of the spinal cord in a mouse spinal cord contusion injury model.
Fig. 23 is a graph showing the area of remaining myelin in a mouse spinal cord contusion injury model.

Description of Embodiments

Next, the present invention will be specifically described.
Unless otherwise specified, the expression "A to B" in a numerical range means a value that is equal to or greater than A and equal to or less than B. In addition,% means mass%.

<Anti-human CD300A monoclonal antibody>

An anti-human CD300A monoclonal antibody or an antigen-binding fragment thereof, which is one aspect of the present invention, satisfies the following requirement (i), (ii), or (iii).

(i) The anti-human CD300A monoclonal antibody or the antigen-binding fragment thereof includes CDR1, CDR2 and CDR3 sequences of a heavy chain variable region having the amino acid sequence of SEQ ID NO: 7, and CDR1, CDR2 and CDR3 sequences of a light chain variable region having the amino acid sequence of SEQ ID NO: 8.
(ii) The anti-human CD300A monoclonal antibody or the antigen-binding fragment thereof includes CDR1, CDR2 and CDR3 sequences of a heavy chain variable region having the amino acid sequence of SEQ ID NO: 15, and CDR1, CDR2 and CDR3 sequences of a light chain variable region having the amino acid sequence of SEQ ID NO: 16.
(iii) The anti-human CD300A monoclonal antibody or the antigen-binding fragment thereof includes CDR1, CDR2 and CDR3 sequences of a heavy chain variable region having the amino acid sequence of SEQ ID NO: 23, and CDR1, CDR2 and CDR3 sequences of a light chain variable region having the amino acid sequence of SEQ ID NO: 24.

Note that the complementarity determining region (CDR) of the heavy chain variable region is also referred to as HCDR, and the CDR of the light chain variable region is also referred to as LCDR.
The anti-human CD300A monoclonal antibody satisfying the requirement (i) is also referred to as an anti-human CD300A monoclonal antibody (i).
The anti-human CD300A monoclonal antibody satisfying the requirement (ii) is also referred to as an anti-human CD300A monoclonal antibody (ii).
The anti-human CD300A monoclonal antibody satisfying the requirement (iii) is also referred to as an anti-human CD300A monoclonal antibody (iii).
The anti-human CD300A monoclonal antibody (i), the anti-human CD300A monoclonal antibody (ii), and the anti-human CD300A monoclonal antibody (iii) are also collectively referred to as an anti-human CD300A monoclonal antibody (X). In addition, when simply referred to as human CD300A, human CD300A-R and human CD300A-R are included.
The anti-human CD300A monoclonal antibody (X) is an antibody that specifically recognizes human CD300A. "Specifically recognizing" means that the antibody binds to human CD300A but does not bind to proteins other than human CD300A, particularly human CD300C.
In the present specification, "binding" means binding with high affinity, that is, binding with $K_D$ (dissociation constant) of preferably $1.0 \times 10^{-5}$ M or less, more preferably $1.0 \times 10^{-6}$ M or less, and still more preferably $1.0 \times 10^{-7}$ M or less.
In the present specification, "does not bind" means substantially no binding, and means either no binding to the antigen protein or no high-affinity binding, that is, binding with $K_D$ of preferably greater than $1 \times 10^{-5}$ M, more preferably $1 \times 10^{-4}$ M or more, still more preferably $1 \times 10^{-3}$ M or more, and particularly preferably $1 \times 10^{-2}$ M or more, as a $K_D$ (dissociation constant) for the antigenic protein
$K_D$ can be measured by a known method, for example, a Bio Layer Interferometry (BLI) method or a Surface Plasmon Resonance (SPR) method, and is preferably measured by the BLI method.
The binding properties of the antibody can be measured by a known method, for example, a method such as immunoprecipitation, Western blotting, enzyme immunoassay (EIA), enzyme-linked immunosorbent assay (ELISA), flow cytometry, or pull-down assay.
Human CD300A includes an intracellular region, a transmembrane region, and an extracellular region. The anti-human CD300A monoclonal antibody (X) is preferably an antibody that binds to all or a part of the extracellular region of human CD300A. The extracellular region of the human CD300A corresponds to amino acids at positions 18 to 178 of the amino acid sequence of human CD300A (299 amino acids).
A class and a subclass of the anti-human CD300A monoclonal antibody (X) are not particularly limited as long as the effect of the present invention is exhibited, may be any of IgG, IgM, IgA, IgD, and IgE, and are preferably IgG, and more preferably IgG1. The heavy chain of the anti-human CD300A monoclonal antibody (X) may be any of $\gamma$, $\mu$, $\alpha$, $\delta$, and $\varepsilon$, and is

preferably γ, and more preferably γ1. The light chain of the anti-human CD300A monoclonal antibody (X) may be any of κ and λ, and is preferably κ.

The anti-human CD300A monoclonal antibody (X) can be produced by a known method. Specifically, immunization can be performed by administering the antigen, optionally together with an adjuvant, one or more times by subcutaneous, intramuscular, intravenous, footpad, or intraperitoneal injection into a mammal, preferably a mouse, rat, hamster, guinea pig, or rabbit. The adjuvant is not particularly limited, and it is possible to use, for example, complete Freund's adjuvant, incomplete Freund's adjuvant, sodium hydroxide, aluminum hydroxide (Alum), calcium phosphate, aluminum phosphate, alum, HEPES, and carboxyvinyl polymer, but complete Freund's adjuvant and incomplete Freund's adjuvant are preferable.

The preparation of hybridomas that secrete monoclonal antibodies can be performed according to the method of Köhler and Milstein (Nature, 1975, Vol. 256, pp. 495-497) or methods based thereon. That is, as described above, a hybridoma can be prepared by cell fusion of antibody-producing cells contained in, for example, the spleen, lymph nodes, bone marrow, or tonsils, preferably the spleen, obtained from an immunized mammal, and myeloma cells having no autoantibody-producing ability, preferably derived from a mammal such as a mouse, rat, guinea pig, hamster, rabbit, or human, and more preferably from a mouse, rat, or human.

As the myeloma cells used for cell fusion, established cells obtained from mice, such as P3-U1, NS-1, SP2/0, 653, X63, and AP-1, can be used.

Screening of hybridoma clones producing monoclonal antibodies can be performed by culturing the hybridomas, for example, in microtiter plates, measuring the reactivity of the culture supernatant from wells showing growth to the antigen used in the above-described mouse immunization by measurement methods such as radioimmunoassay (RIA), ELISA, or fluorescence-assisted cell sorting (FACS), and selecting clones that produce monoclonal antibodies that specifically bind to the antigen.

The production of the monoclonal antibodies from the selected hybridoma clones can be performed by culturing the hybridomas in vitro, or culturing the hybridomas in ascites of, for example, a mouse, a rat, a guinea pig, a hamster, or a rabbit, preferably a mouse or a rat, and more preferably a mouse, and isolating the monoclonal antibodies from the obtained culture supernatant or ascites of the mammal.

As the anti-human CD300A monoclonal antibody (X), a recombinant antibody obtained by cloning an antibody gene from an antibody-producing cell, for example, a hybridoma, incorporating the antibody gene into an appropriate vector, introducing the vector into a host, and producing the antibody using a genetic recombination technique may be used.

Specifically, mRNA encoding the variable region of the antibody can be isolated from a hybridoma producing a desired antibody or an immune cell producing an antibody, for example, a sensitized lymphocyte, which is immortalized by, for example, an oncogene. cDNA of the antibody variable region can be synthesized from the obtained mRNA using a reverse transcriptase and incorporated into an expression vector.

When DNA encoding the variable region of the desired antibody is obtained, it can be ligated to DNA encoding a desired antibody constant region and incorporated into an expression vector. Alternatively, the DNA encoding the variable region of the antibody may be incorporated into an expression vector containing DNA of the antibody constant region. In order to produce a desired antibody, the antibody gene is incorporated into an expression vector so as to be expressed under the control of an expression control region, such as an enhancer/promoter. Next, the host cell can be transformed with the expression vector to express the antibody.

The expression of the antibody gene may be performed by incorporating DNA encoding either the heavy chain or the light chain separately into individual expression vectors and simultaneously transforming the host, or by incorporating DNA encoding both the heavy and light chains into a single expression vector and transforming the host.

As a method for producing the antibody, a so-called phage display method (Nature Biotechnology 23, 1105 (2005)) may be used.

As the anti-human CD300A monoclonal antibody (X), a culture supernatant of a hybridoma, ascites of a mammal, or a culture supernatant of a transformed host, each containing a monoclonal antibody, may be used as it is, or may be used after the monoclonal antibody is isolated and purified. The purification can be performed, for example, by subjecting, for example, culture supernatant or ascites to saturated ammonium sulfate, ion-exchange chromatography (for example, DEAE or DE52), or affinity column chromatography such as anti-immunoglobulin columns, protein A columns, or protein G columns.

The anti-human CD300A monoclonal antibody (X) may be, for example, a mouse antibody, a rat antibody, a guinea pig antibody, a hamster antibody, a rabbit antibody, a monkey antibody, or a dog antibody, and may also be a recombinant antibody such as an antibody containing a human Fc region, an antibody containing a human constant region, a human-type chimeric antibody (hereinafter, simply referred to as a chimeric antibody), a humanized antibody (also referred to as a human-type CDR-grafted antibody), or a human antibody, in order to reduce immunogenicity in humans.

A chimeric antibody is an antibody including a heavy chain variable region (hereinafter, referred to as VH) and a light chain variable region (hereinafter, referred to as VL) of a non-human animal antibody, and a heavy chain constant region (hereinafter, referred to as CH) and a light chain constant region (hereinafter, referred to as CL) of a human antibody. The type of animal for the variable region is not particularly limited as long as it is an animal capable of producing hybridomas, such as a mouse, a rat, a hamster, or a rabbit.

A human-type chimeric anti-human CD300A antibody can be produced by obtaining cDNAs encoding the VH and VL of a non-human animal antibody that specifically binds to human CD300A, inserting the cDNAs into each of expression vectors having genes encoding the CH and CL of a human antibody to construct a human-type chimeric antibody expression vector, and introducing the expression vector into animal cells to express the antibody.

The CH of the human-type chimeric antibody is not particularly limited as long as it is a human immunoglobulin (hereinafter, also referred to as hIg), and is preferably of hIgG class. The CL of the human-type chimeric antibody is not particularly limited as long as it belongs to hIgG.

A humanized antibody is an antibody in which the CDRs of the VH and VL of a non-human animal antibody are grafted into appropriate positions of the VH and VL of a human antibody. The anti-human CD300A humanized antibody can be produced by constructing cDNAs encoding variable regions (hereinafter also referred to as V regions) in which the CDRs of the VH and VL of a non-human animal antibody that specifically binds to human CD300A are grafted into frameworks (hereinafter also referred to as FRs) of the VH and VL of arbitrary human antibody, respectively, inserting the cDNAs into expression vectors having DNAs encoding the CH and CL of a human antibody to construct humanized antibody expression vectors, and introducing the expression vectors into animal cells for expression. The amino acid sequences of the FRs of the VH and VL of the human antibody are not particularly limited as long as they are amino acid sequences derived from a human antibody. The CH of the humanized antibody is not particularly limited as long as it is hIg, but a hIgG class is preferable. The CL of the humanized antibody is not particularly limited as long as it belongs to hIg.

The anti-human CD300A monoclonal antibody (X) may be, for example, a multi-specific antibody, a recycling antibody, a sweeping antibody, or a conjugate antibody. In addition, the anti-human CD300A monoclonal antibody (X) may chemically or genetically bind to functional molecules such as a non-peptide polymer such as polyethylene glycol (PEG), a radioactive substance, a toxin, a small molecule compound, a cytokine, a growth factor, albumin, an enzyme, and other antibodies. These antibodies can be produced by a known method.

The anti-human CD300A monoclonal antibodies (X) may be used alone or in combination of two or more kinds thereof.

<Antigen-binding fragment>

An antigen-binding fragment of the anti-human CD300A monoclonal antibody (X) refers to a protein containing a part of the anti-human CD300A monoclonal antibody (X), which is capable of binding to the antigen, which is human CD300A. Examples of the antigen-binding fragment include F(ab')2, Fab', Fab, disulfide bond stabilized Fv (dsFv), a single chain antibody (scFv), a diabody, and a polymer thereof.

The antigen-binding fragment may chemically or genetically bind to functional molecules such as a non-peptide polymer such as polyethylene glycol (PEG), a radioactive substance, a toxin, a small molecule compound, a cytokine, a growth factor, albumin, an enzyme, and other antibodies.

The antigen-binding fragments may be used alone or in combination of two or more kinds thereof. In addition, the anti-human CD300A monoclonal antibody (X) and the antigen-binding fragment thereof may also be used in combination.

[Anti-human CD300A monoclonal antibody (i)]

The anti-human CD300A monoclonal antibody (i) includes CDR1, CDR2 and CDR3 sequences of a heavy chain variable region having the amino acid sequence of SEQ ID NO: 7, and CDR1, CDR2 and CDR3 sequences of a light chain variable region having the amino acid sequence of SEQ ID NO: 8.

The CDR1 to CDR3 sequences in the heavy chain variable region may be determined based on the amino acid sequence of SEQ ID NO: 7, and the CDR1 to CDR3 sequences in the light chain variable region may be determined based on the amino acid sequence of SEQ ID NO: 8.

The method for determining the CDR1 to CDR3 sequences in the heavy chain variable region or the light chain variable region is not particularly limited, and known methods such as Kabat, Chothia, Aho, and IMGT can be used. Among them, the method of Kabat is preferable because it is most widely used.

The amino acid sequence of the anti-human CD300A monoclonal antibody (i) is not particularly limited in regions other than HCDR1 to HCDR3 and LCDR1 to LCDR3, as long as the effect of the present invention is exhibited.

The anti-human CD300A monoclonal antibody (i) binds to human CD300A-R and human CD300A-Q, does not bind to human CD300C, and has an activity (neutralizing activity) of inhibiting binding between human CD300A-R and a ligand thereof, and binding between human CD300A-Q and a ligand thereof.

The anti-human CD300A monoclonal antibody (i) is preferably an antibody that inhibits or reduces signal transduction mediated by the ITIM of human CD300A.

The ligand of human CD300A is not particularly limited as long as it is a molecule reported to bind to human CD300A, but is preferably a molecule exposed on the surface of dead cells or activated cells, and more preferably a lipid exposed on the surface of dead cells or activated cells. More specifically, the ligand of human CD300A is preferably phosphatidylserine (PS), phosphatidylethanolamine (PE), and more preferably PS.

A method for measuring an activity (neutralizing activity) of inhibiting binding between human CD300A and a ligand thereof is not particularly limited, and can be performed by a known method, and examples of the known method include a method of analyzing, by flow cytometry, a degree of inhibition of binding between a human CD300A recombinant protein and a cell induced to undergo apoptosis.

The anti-human CD300A monoclonal antibody (i) preferably includes CDR1, CDR2, and CDR3 sequences of the heavy chain variable region which are the amino acid sequence of SEQ ID NO: 1, the amino acid sequence of SEQ ID NO: 2, and the amino acid sequence of SEQ ID NO: 3, respectively, and

CDR1, CDR2, and CDR3 sequences of the light chain variable region which are the amino acid sequence of SEQ ID NO: 4, the amino acid sequence of SEQ ID NO: 5, and the amino acid sequence of SEQ ID NO: 6, respectively.

The anti-human CD300A monoclonal antibody (i) more preferably includes a heavy chain variable region having the amino acid sequence of SEQ ID NO: 1 as HCDR1, the amino acid sequence of SEQ ID NO: 2 as HCDR2, and the amino acid sequence of SEQ ID NO: 3 as HCDR3, and

a light chain variable region having the amino acid sequence of SEQ ID NO: 4 as LCDR1, the amino acid sequence of SEQ ID NO: 5 as LCDR2, and the amino acid sequence of SEQ ID NO: 6 as LCDR3.

The anti-human CD300A monoclonal antibody (i) preferably includes a heavy chain variable region having an amino acid sequence that is at least 90%, 93%, 95%, 96%, 97%, 98%, or 99% identical to the amino acid sequence of SEQ ID NO: 31, and

a light chain variable region having an amino acid sequence that is at least 90%, 93%, 95%, 96%, 97%, 98%, or 99% identical to the amino acid sequence of SEQ ID NO: 32.

Note that, in the present specification, the percentage of sequence identity is determined by the sequence of the antibody maximally aligned according to Kabat numbering convention. After alignment, when comparing the target antibody region (for example, the light chain variable region) with the same region of a control antibody, the percentage of sequence identity between the target antibody region and the control antibody region is calculated by dividing the number of positions occupied by the same amino acid in both the target antibody region and the control antibody region by the total number of aligned positions in the two regions (gaps not counted) and multiplying by 100.

The anti-human CD300A monoclonal antibody (i) may be an antibody including a heavy chain variable region having an amino acid sequence that is at least 95% identical to SEQ ID NO: 31 and has 1 to 6 mutations of amino acid residues in a region other than HCDR1 to HCDR3 in the amino acid sequence of SEQ ID NO: 31, and a light chain variable region having an amino acid sequence that is at least 95% identical to SEQ ID NO: 32 and has 1 to 6 mutations of amino acid residues in a region other than LCDR1 to LCDR3 in the amino acid sequence of SEQ ID NO: 32.

The anti-human CD300A monoclonal antibody (i) may be an antibody including a heavy chain variable region having an amino acid sequence that is at least 97% identical to SEQ ID NO: 31 and has 1 to 4 mutations of amino acid residues in a region other than HCDR1 to HCDR3 in the amino acid sequence of SEQ ID NO: 31, and a light chain variable region having an amino acid sequence that is at least 97% identical to SEQ ID NO: 32 and has 1 to 3 mutations of amino acid residues in a region other than LCDR1 to LCDR3 in the amino acid sequence of SEQ ID NO: 32.

In the present specification, the mutation of the amino acid residue means substitution, insertion, or deletion of a single amino acid residue. Regarding the mutation of the amino acid residue, substitution is preferable among substitution, insertion, and deletion, and conservative substitution is more preferable.

In the present specification, the "conservative substitution" is substitution of an amino acid residue with another chemically similar amino acid residue so as not to substantially alter the activity of the peptide. For example, examples thereof include a case in which a certain hydrophobic residue is substituted with another hydrophobic residue, and a case in which a certain polar residue is substituted with another polar residue having the same charge. Examples of chemically similar amino acids for which such conservative substitution can be performed include alanine, valine, isoleucine, leucine, proline, tryptophan, phenylalanine, and methionine as non-polar (hydrophobic) amino acids. Examples of a polar (neutral) amino acid include glycine, serine, threonine, tyrosine, glutamine, asparagine, and cysteine. Examples of a positively charged (basic) amino acid include arginine, histidine, and lysine. In addition, examples of a negatively charged (acidic) amino acid include aspartic acid and glutamic acid.

The anti-human CD300A monoclonal antibody (i) is preferably a humanized antibody because it can be administered to a human.

When the anti-human CD300A monoclonal antibody (i) is a humanized antibody, the anti-human CD300A monoclonal antibody (i) may preferably have back mutations of amino acid residues in regions other than HCDR1 to HCDR3 of the heavy chain variable region and/or regions other than LCDR1 to LCDR3 of the light chain variable region.

The back mutation of the amino acid residue refers to a substitution of a single amino acid residue found in a human antibody framework for a corresponding amino acid residue found in a mouse antibody framework. Both low immunogenicity and high binding affinity to the antigen are easily achieved by an appropriate back mutation of amino acid residues.

The anti-human CD300A monoclonal antibody (i) preferably has 1 to 3, and more preferably 1 or 2 back mutations of amino acid residues in regions other than HCDR1 to HCDR3 of the heavy chain variable region, and preferably has 1 to 5, and more preferably 1 to 4 back mutations of amino acid residues in regions other than LCDR1 to LCDR3 of the light chain variable region.

Amino acid residues to be subjected to back mutation can be selected from amino acid residues within the variable region that bind to the antigen, amino acid residues adjacent to CDR regions, amino acid residues that interact with CDR regions, amino acid residues involved in the formation of the antigen-binding site, or amino acid residues involved in the VL-VH interface.

From the viewpoint of binding affinity to human CD300A, the position of the back mutation in the heavy chain variable region of the anti-human CD300A monoclonal antibody (i) is at least one selected from the group consisting of G at position 44, G at position 10, R at position 19, G at position 42, and V at position 89, more preferably G at position 44, still more preferably a combination of G at position 44 and at least one selected from the group consisting of G at position 10, R at position 19, G at position 42, and V at position 89, and particularly preferably a combination of G at position 44 and G at position 42.

From the viewpoint of binding affinity to human CD300A, the back mutation in the heavy chain variable region of the anti-human CD300A monoclonal antibody (i) is preferably at least one selected from the group consisting of G44R, G10D, R19K, G42D, and V89M, more preferably G44R, still more preferably a combination of G44R and at least one selected from the group consisting of G10D, R19K, G42D, and V89M, and particularly preferably a combination of G44R and G42D.

Note that, in the present specification, the amino acid residue at position X refers to the amino acid residue located at position X from the N-terminus of the protein without including the signal peptide. In addition, in the description such as G44R, the letter on the left indicates the amino acid residue before mutation, the number in the center indicates the position of the amino acid residue, and the letter on the right indicates the amino acid residue after mutation.

From the perspective of binding affinity to human CD300A, the position of the back mutations in the light chain variable region of the anti-human CD300A monoclonal antibody (i) is preferably at least one selected from the group consisting of L at position 47, F at position 71, D at position 1, V at position 3, M at position 4, I at position 21, K at position 42, and D at position 70, more preferably L at position 47, still more preferably L at position 47 and F at position 71, even more preferably a combination of L at position 47 and F at position 71 and at least one selected from the group consisting of D at position 1, V at position 3, M at position 4, I at position 21, K at position 42, and D at position 70, particularly preferably a combination of L at position 47, F at position 71, and D at position 1 or I at position 21, and further even more preferably a combination of L at position 47, F at position 71, D at position 1, and I at position 21.

From the perspective of binding affinity to human CD300A, the back mutation in the light chain variable region of the anti-human CD300A monoclonal antibody (i) is preferably at least one selected from the group consisting of L47W, F71Y, D1Q, Q3V, M4L, I21M, K42S, and D70S, more preferably L47W, still more preferably L47W and F71Y, particularly preferably a combination of L47W and F71Y and at least one selected from the group consisting of D1Q, Q3V, M4L, I21M, K42S, and D70S, even more preferably a combination of L47W, F71Y, and D1Q or I21M, and further even more preferably a combination of L47W, F71Y, D1Q, and I21M.

The anti-human CD300A monoclonal antibody (i) preferably includes a heavy chain variable region having the amino acid sequence of SEQ ID NO: 31 and a light chain variable region having the amino acid sequence of SEQ ID NO: 32.

The anti-human CD300A monoclonal antibody (i) preferably contains a mutation of an amino acid residue that eliminates the effector function of an IgG antibody.

The mutation of the amino acid residue for eliminating the effector function of the IgG antibody is, for example, a mutation in which a branched chain amino acid such as valine (V), leucine (L), or isoleucine (I), a hydrophobic amino acid such as proline (P), methionine (M), or tryptophan (W), or an amino acid phosphorylated and involved in signal transduction, such as tyrosine (Y), serine (S), or threonine (T), is substituted with alanine (A) or phenylalanine (F) in the constant region of the heavy chain, and is preferably a mutation in which L is substituted with A or F, and more preferably a mutation in which two consecutive L are substituted with AA, FF, AF, or FA.

In the anti-human CD300A monoclonal antibody (i), amino acid residues at positions 234 and 235 of the heavy chain are preferably A or F, and more preferably A at both positions. Such an anti-human CD300A monoclonal antibody (i) is useful for treatment of human diseases because it eliminates the effector function of the IgG antibody and hardly aggregates platelets.

The anti-human CD300A monoclonal antibody (i) preferably includes a heavy chain having an amino acid sequence that is 80% or more, 85% or more, 90% or more, 93% or more, 95% or more, 97% or more, 98% or more, or 99% or more identical to the amino acid sequence of SEQ ID NO: 35, and a light chain having an amino acid sequence that is 80% or more, 85% or more, 90% or more, 93% or more, 95% or more, 97% or more, 98% or more, or 99% or more identical to the amino acid sequence of SEQ ID NO: 36.

The anti-human CD300A monoclonal antibody (i) preferably includes a heavy chain having the amino acid sequence of SEQ ID NO: 35 and a light chain having the amino acid sequence of SEQ ID NO: 36.

The anti-human CD300A monoclonal antibody (i) binds to both human CD300A-R and human CD300A-Q, does not bind to human CD300C, and inhibits binding between human CD300A-R and a ligand thereof as well as binding between human CD300A-Q and a ligand thereof, and therefore has the following advantages.

· Even when both CD300A and CD300C are expressed on the cell surface, the function of CD300A can be examined while excluding the possibility that the anti-human CD300A monoclonal antibody (i) binds to CD300C.

· When the anti-human CD300A monoclonal antibody (i) is administered to a human, side effects that may arise from the antibody binding to CD300C can be avoided. Since human CD300A is an inhibitory type and human CD300C is conversely an activating type, the side effects refer to, for example, the cancellation of the inhibitory signal of human CD300A, which may be caused by the antibody binding to CD300C, and the resulting effects. In addition, since the function of CD300C has not been well analyzed, the side effects refer to, for example, effects caused by unknown functions of CD300C.

· When the anti-human CD300A monoclonal antibody (i) is administered to a human, both a human having the arginine-type CD300A and a human having the glutamine-type CD300A can be subjects for administration. When an antibody targeting a protein with a non-synonymous single nucleotide polymorphism is used as a pharmaceutical, the development of the pharmaceutical requires a great deal of cost and time, and therefore, when the antibody reacts with all variants, a single antibody can be administered to many humans, and thus there is a great economic merit for development. In addition, it is not necessary to determine which variant is present before administration.

· It is effective for the treatment or prevention of diseases caused by the function of human CD300A.

· It is effective for the treatment or prevention of diseases associated with the function of human CD300A.

[Anti-human CD300A monoclonal antibody (ii)]

The anti-human CD300A monoclonal antibody (ii) includes CDR1, CDR2 and CDR3 sequences of a heavy chain variable region having the amino acid sequence of SEQ ID NO: 15, and CDR1, CDR2 and CDR3 sequences of a light chain variable region having the amino acid sequence of SEQ ID NO: 16.

The CDR1 to CDR3 sequences in the heavy chain variable region may be determined based on the amino acid sequence of SEQ ID NO: 15, and the CDR1 to CDR3 sequences in the light chain variable region may be determined based on the amino acid sequence of SEQ ID NO: 16.

The method for determining the CDR sequences in the heavy chain variable region or the light chain variable region is not particularly limited, and known methods such as Kabat, Chothia, Aho, and IMGT can be used. Among them, the method of Kabat is preferable because it is most widely used.

The amino acid sequence of the anti-human CD300A monoclonal antibody (ii) is not particularly limited in regions other than HCDR1 to HCDR3 and LCDR1 to LCDR3, as long as the effect of the present invention is exhibited.

The anti-human CD300A monoclonal antibody (ii) binds to human CD300A-R, does not bind to human CD300A-Q or human CD300C, and has an activity (neutralizing activity) of inhibiting binding between human CD300A-R and a ligand thereof.

The anti-human CD300A monoclonal antibody (ii) is preferably an antibody that inhibits or reduces signal transduction mediated by the ITIM of human CD300A-R.

The ligand of human CD300A-R is not particularly limited as long as it is a molecule reported to bind to human CD300A-R, but is preferably a molecule exposed on the surface of dead cells or activated cells, and more preferably a lipid exposed on the surface of dead cells or activated cells. More specifically, the ligand of human CD300A-R is preferably phosphatidylserine (PS), phosphatidylethanolamine (PE), and more preferably PS.

A method for measuring an activity (neutralizing activity) of inhibiting binding between human CD300A-R and a ligand thereof is not particularly limited, and can be performed by a known method, and examples of the known method include a method of analyzing, by flow cytometry, a degree of inhibition of binding between a human CD300A-R recombinant protein and a cell induced to undergo apoptosis.

The anti-human CD300A monoclonal antibody (ii) preferably includes CDR1, CDR2, and CDR3 sequences of the heavy chain variable region which are the amino acid sequence of SEQ ID NO: 9, the amino acid sequence of SEQ ID NO: 10, and the amino acid sequence of SEQ ID NO: 11, respectively, and CDR1, CDR2, and CDR3 sequences of the light chain variable region which are the amino acid sequence of SEQ ID NO: 12, the amino acid sequence of SEQ ID NO: 13, and the amino acid sequence of SEQ ID NO: 14, respectively.

The anti-human CD300A monoclonal antibody (ii) more preferably includes a heavy chain variable region having the amino acid sequence of SEQ ID NO: 9 as HCDR1, the amino acid sequence of SEQ ID NO: 10 as HCDR2, and the amino acid sequence of SEQ ID NO: 11 as HCDR3; and

a light chain variable region having the amino acid sequence of SEQ ID NO: 12 as LCDR1, the amino acid sequence of SEQ ID NO: 13 as LCDR2, and the amino acid sequence of SEQ ID NO: 14 as LCDR3.

The anti-human CD300A monoclonal antibody (ii) preferably includes a heavy chain variable region having an amino acid sequence that is at least 80%, 85%, 90%, 93%, 95%, 96%, 97%, 98%, or 99% identical to the amino acid sequence of SEQ ID NO: 15, and a light chain variable region having an amino acid sequence that is at least 80%, 85%, 90%, 93%, 95%, 96%, 97%, 98%, or 99% identical to the amino acid sequence of SEQ ID NO: 16.

The anti-human CD300A monoclonal antibody (ii) may be an antibody including a heavy chain variable region having an amino acid sequence that is at least 80% identical to SEQ ID NO: 15 and has one or more mutations of amino acid residues in a region other than HCDR1 to HCDR3 in the amino acid sequence of SEQ ID NO: 15, and a light chain variable region having an amino acid sequence that is at least 80% identical to SEQ ID NO: 16 and has one or more mutations of amino acid residues in a region other than LCDR1 to LCDR3 in the amino acid sequence of SEQ ID NO: 16.

The anti-human CD300A monoclonal antibody (ii) preferably includes a heavy chain variable region having the amino acid sequence of SEQ ID NO: 15 and a light chain variable region having the amino acid sequence of SEQ ID NO: 16.

The anti-human CD300A monoclonal antibody (ii) binds to human CD300A-R, does not bind to human CD300A-Q or human CD300C, and has an activity (neutralizing activity) of inhibiting binding between human CD300A-R and a ligand thereof, thereby providing the following advantages.

· Even when both CD300A-R and CD300C are expressed on the cell surface, the function of CD300A-R can be examined while excluding the possibility that the anti-human CD300A monoclonal antibody (ii) binds to CD300C.

· When the anti-human CD300A monoclonal antibody (ii) is administered to a human, side effects that may arise from the antibody binding to CD300C can be avoided. Since human CD300A-R is an inhibitory type and human CD300C is conversely an activating type, the side effects refer to, for example, the cancellation of the inhibitory signal of human CD300A-R, which may be caused by the antibody binding to CD300C, and the resulting effects. In addition, since the function of CD300C has not been well analyzed, the side effects refer to, for example, effects caused by unknown functions of CD300C.

· It is effective for the treatment or prevention of diseases caused by the function of human CD300A-R.

· It is effective for the treatment or prevention of diseases associated with the function of human CD300A-R.

· Using the anti-human CD300A monoclonal antibody (ii), it can be determined whether CD300A is of an arginine-type or a glutamine-type.

[Anti-human CD300A monoclonal antibody (iii)]

The anti-human CD300A monoclonal antibody (iii) includes CDR1, CDR2 and CDR3 sequences of a heavy chain variable region having the amino acid sequence of SEQ ID NO: 23, and CDR1, CDR2 and CDR3 sequences of a light chain variable region having the amino acid sequence of SEQ ID NO: 24.

The CDR1 to CDR3 sequences in the heavy chain variable region may be determined based on the amino acid sequence of SEQ ID NO: 23, and the CDR1 to CDR3 sequences in the light chain variable region may be determined based on the amino acid sequence of SEQ ID NO: 24.

The method for determining the CDR sequences in the heavy chain variable region or the light chain variable region is not particularly limited, and known methods such as Kabat, Chothia, Aho, and IMGT can be used. Among them, the method of Kabat is preferable because it is most widely used.

The amino acid sequence of the anti-human CD300A monoclonal antibody (iii) is not particularly limited in regions other than HCDR1 to HCDR3 and LCDR1 to LCDR3, as long as the effect of the present invention is exhibited.

The anti-human CD300A monoclonal antibody (iii) binds to human CD300A-Q, does not bind to human CD300A-R or human CD300C, and has an activity (neutralizing activity) of inhibiting binding between human CD300A-Q and a ligand thereof.

The anti-human CD300A monoclonal antibody (iii) is preferably an antibody that inhibits or reduces signal transduction mediated by the ITIM of human CD300A-Q.

The ligand of human CD300A-Q is not particularly limited as long as it is a molecule reported to bind to human CD300A-Q, but is preferably a molecule exposed on the surface of dead cells or activated cells, and more preferably a lipid exposed on the surface of dead cells or activated cells. More specifically, the ligand of human CD300A-Q is preferably phosphatidylserine (PS), phosphatidylethanolamine (PE), and more preferably PS.

A method for measuring an activity (neutralizing activity) of inhibiting binding between human CD300A-Q and a ligand thereof is not particularly limited, and can be performed by a known method, and examples of the known method include a method of analyzing, by flow cytometry, a degree of inhibition of binding between a human CD300A-Q recombinant protein and a cell induced to undergo apoptosis.

The anti-human CD300A monoclonal antibody (iii) preferably includes CDR1, CDR2, and CDR3 sequences of the heavy chain variable region which are the amino acid sequence of SEQ ID NO: 17, the amino acid sequence of SEQ ID NO: 18, and the amino acid sequence of SEQ ID NO: 19, respectively, and

CDR1, CDR2, and CDR3 sequences of the light chain variable region which are the amino acid sequence of SEQ ID NO: 20, the amino acid sequence of SEQ ID NO: 21, and the amino acid sequence of SEQ ID NO: 22, respectively.

The anti-human CD300A monoclonal antibody (iii) more preferably includes a heavy chain variable region having the amino acid sequence of SEQ ID NO: 17 as HCDR1, the amino acid sequence of SEQ ID NO: 18 as HCDR2, and the amino acid sequence of SEQ ID NO: 19 as HCDR3; and

a light chain variable region having the amino acid sequence of SEQ ID NO: 20 as LCDR1, the amino acid sequence of SEQ ID NO: 21 as LCDR2, and the amino acid sequence of SEQ ID NO: 22 as LCDR3.

The anti-human CD300A monoclonal antibody (iii) preferably includes a heavy chain variable region having an amino acid sequence that is at least 80%, 85%, 90%, 93%, 95%, 96%, 97%, 98%, or 99% identical to the amino acid sequence of SEQ ID NO: 23, and a light chain variable region having an amino acid sequence that is at least 80%, 85%, 90%, 93%, 95%, 96%, 97%, 98%, or 99% identical to the amino acid sequence of SEQ ID NO: 24.

The anti-human CD300A monoclonal antibody (iii) may be an antibody including a heavy chain variable region having an amino acid sequence that is at least 80% identical to SEQ ID NO: 23 and has one or more mutations of amino acid residues in a region other than HCDR1 to HCDR3 in the amino acid sequence of SEQ ID NO: 23, and a light chain variable region having an amino acid sequence that is at least 80% identical to SEQ ID NO: 24 and has one or more mutations of amino acid residues in a region other than LCDR1 to LCDR3 in the amino acid sequence of SEQ ID NO: 24.

The anti-human CD300A monoclonal antibody (iii) preferably includes a heavy chain variable region having the amino acid sequence of SEQ ID NO: 23 and a light chain variable region having the amino acid sequence of SEQ ID NO: 24.

The anti-human CD300A monoclonal antibody (iii) binds to human CD300A-Q, does not bind to human CD300A-R or human CD300C, and has an activity (neutralizing activity) of inhibiting binding between human CD300A-Q and a ligand thereof, thereby providing the following advantages.

· Even when both CD300A-Q and CD300C are expressed on the cell surface, the function of CD300A-Q can be examined while excluding the possibility that the anti-human CD300A monoclonal antibody (iii) binds to CD300C.
· When the anti-human CD300A monoclonal antibody (iii) is administered to a human, side effects that may arise from the antibody binding to CD300C can be avoided. Since human CD300A-Q is an inhibitory type and human CD300C is conversely an activating type, the side effects refer to, for example, the cancellation of the inhibitory signal of human CD300A-Q, which may be caused by the antibody binding to CD300C, and the resulting effects. In addition, since the function of CD300C has not been well analyzed, the side effects refer to, for example, effects caused by unknown functions of CD300C.
· It is effective for the treatment or prevention of diseases caused by the function of human CD300A-Q.
· It is effective for the treatment or prevention of diseases associated with the function of human CD300A-Q.
· Using the anti-human CD300A monoclonal antibody (iii), it can be determined whether CD300A is of an arginine-type or a glutamine-type.

<Pharmaceutical composition>

A pharmaceutical composition according to one aspect of the present invention is for treatment or prevention of a disease caused by a function of human CD300A and contains an anti-human CD300A monoclonal antibody (X) or an antigen-binding fragment thereof. The disease caused by the function of human CD300A refers to a disease that is directly or indirectly developed by the function of human CD300A.

A pharmaceutical composition according to one aspect of the present invention is for treatment or prevention of a disease associated with a function of human CD300A, and contains an anti-human CD300A monoclonal antibody (X) or an antigen-binding fragment thereof. The disease associated with the function of human CD300A refers to a disease that is caused, exacerbated, or progressed directly or indirectly by the function of human CD300A.

The pharmaceutical composition may be any pharmaceutical composition containing an anti-human CD300A monoclonal antibody (X) or an antigen-binding fragment thereof as an active ingredient, but it is preferably provided as a pharmaceutical formulation prepared by mixing with one or more pharmaceutically acceptable carriers by any method well known in the field of pharmaceutical formulation.

The pharmaceutical composition is preferably for treatment or prevention of a disease caused by a function of human CD300A, and contains the anti-human CD300A monoclonal antibody (i) or the antigen-binding fragment thereof.

The pharmaceutical composition is preferably for treatment or prevention of a disease associated with a function of human CD300A, and contains the anti-human CD300A monoclonal antibody (i) or the antigen-binding fragment thereof.

The disease caused by the function of human CD300A is not limited as long as it is caused by the function of human CD300A. The disease caused by the function of the human CD300A is preferably at least one selected from the group consisting of ischemic diseases, inflammatory diseases, infectious diseases, allergic diseases, organ fibrosis, and autoimmune diseases. The disease caused by the function of the human CD300A is preferably spinal cord injury.

The disease associated with the function of human CD300A is not particularly limited as long as it is associated with the function of human CD300A. The disease associated with the function of human CD300A is preferably at least one selected from the group consisting of ischemic diseases, inflammatory diseases, infectious diseases, allergic diseases, organ fibrosis, autoimmune diseases, and spinal cord injury. The disease associated with the function of the human CD300A is preferably spinal cord injury.

Note that the term "treatment" includes not only curing a disease or symptom and alleviating (ameliorating) the disease or symptom, and the term "prevention" includes not only preventing the onset of a disease or symptom, but also preventing recurrence of the disease or symptom after curing.

The ischemic disease is not particularly limited, and examples thereof include ischemic heart diseases such as angina pectoris and myocardial infarction; ischemic cerebrovascular diseases such as cerebral infarction and moyamoya disease; ischemic renal diseases such as renal failure; ischemic intestinal diseases such as ischemic colitis and mesenteric artery occlusion; limb ischemia such as gangrene and arteriosclerosis obliterans; retinal ischemia such as diabetic retinopathy; ischemic pulmonary disease; spinal cord ischemia; and conditions exhibiting signs thereof.

The infectious disease is not particularly limited, and examples thereof include bacterial peritonitis and sepsis.

The inflammatory diseases, allergic diseases, organ fibrosis, and autoimmune diseases are not particularly limited, and examples thereof include inflammatory bowel diseases such as ulcerative colitis, Crohn's disease, and ulcerative colitis of the colon; allergic diseases such as celiac disease, atopic dermatitis, bronchial asthma, and food allergies; organ fibrosis of the lungs, kidneys, liver, skin, or heart muscle; and autoimmune diseases such as rheumatoid arthritis, systemic lupus erythematosus, multiple sclerosis, and nephritis.

The spinal cord injury is not particularly limited, but is usually secondary injury. The mode of occurrence of the spinal cord injury is not particularly limited and may be any of a transection injury, a contusion injury, or a penetrating injury, but is preferably a contusion injury. The spinal cord injury may be in any of an acute phase, a subacute phase, and a chronic phase, but is preferably in the acute phase.

<Diagnostic agent>

A diagnostic agent according to one aspect of the present invention contains an anti-human CD300A monoclonal antibody (ii) or an anti-human CD300A monoclonal antibody (iii), or an antigen-binding fragment thereof.

From the viewpoint of accuracy of determination, the diagnostic agent preferably contains an anti-human CD300A monoclonal antibody (ii) or an antigen-binding fragment thereof and an anti-human CD300A monoclonal antibody (iii) or an antigen-binding fragment thereof, and more preferably contains an anti-human CD300A monoclonal antibody (i) or an antigen-binding fragment thereof, an anti-human CD300A monoclonal antibody (ii) or an antigen-binding fragment thereof, and an anti-human CD300A monoclonal antibody (iii) or an antigen-binding fragment thereof.

The diagnostic agent can be used to determine whether CD300A is of the arginine-type or the glutamine-type.

For example, in a test using the anti-human CD300A monoclonal antibody (ii), a positive result indicates that CD300A is the arginine-type, and in a test using the anti-human CD300A monoclonal antibody (iii), a positive result indicates that CD300A is the glutamine-type.

When the diagnostic agent contains the anti-human CD300A monoclonal antibody (ii) and the anti-human CD300A monoclonal antibody (iii), a positive result in a test using the anti-human CD300A monoclonal antibody (ii) and a negative result in a test using the anti-human CD300A monoclonal antibody (iii) indicate that CD300A is the arginine-type, and a negative result in a test using the anti-human CD300A monoclonal antibody (ii) and a positive result in a test using the anti-human CD300A monoclonal antibody (iii) indicate that CD300A is the glutamine-type.

When the diagnostic agent contains the anti-human CD300A monoclonal antibody (i), the anti-human CD300A monoclonal antibody (ii), and the anti-human CD300A monoclonal antibody (iii), a positive result in a test using the anti-human CD300A monoclonal antibody (i), a positive result in a test using the anti-human CD300A monoclonal antibody (ii), and a negative result in a test using the anti-human CD300A monoclonal antibody (iii) indicate that CD300A is the arginine-type, and a positive result in a test using the anti-human CD300A monoclonal antibody (i), a negative result in a test using the anti-human CD300A monoclonal antibody (ii), and a positive result in a test using the anti-human CD300A monoclonal antibody (iii) indicate that CD300A is the glutamine-type.

The test is not particularly limited as long as it is a test method utilizing an antigen-antibody reaction of the antibody, and examples of known test methods include immunochromatography, immunoprecipitation, immunoblotting, enzyme immunoassay (EIA), enzyme-linked immunosorbent assay (ELISA), flow cytometry, and pull-down assay.

The test sample used in the test is not particularly limited as long as it contains human CD300A as an antigen. Examples of the test sample include immune cells such as myeloid cells, NK cells, T cells, and B cells, or tissues, organs, and body fluids containing these cells, and the test sample is preferably blood, and more preferably PBMC.

The diagnostic agent is useful because it enables the determination of whether CD300A is the arginine-type or the glutamine-type through a simple operation using antibodies, without performing genotyping that involves gene extraction and genetic analysis from the test sample.

<Nucleic acid>

One aspect of the present invention is a nucleic acid encoding the anti-human CD300A monoclonal antibody (X) or the antigen-binding fragment thereof.

Examples of the nucleic acid include a nucleic acid encoding the heavy chain variable region of the anti-human CD300A monoclonal antibody (X), a nucleic acid encoding the light chain variable region of the anti-human CD300A monoclonal antibody (X), a nucleic acid encoding the variable region and a portion of the constant region of the heavy chain of the anti-human CD300A monoclonal antibody (X), a nucleic acid encoding the variable region and a portion of the constant region of the light chain of the anti-human CD300A monoclonal antibody (X), a nucleic acid encoding the full-length heavy chain of the anti-human CD300A monoclonal antibody (X), a nucleic acid encoding the full-length light chain of the anti-human CD300A monoclonal antibody (X), and a nucleic acid encoding an scFv in which the heavy chain variable region and the light chain variable region of the anti-human CD300A monoclonal antibody (X) are linked by an appropriate linker.

The nucleic acid can be produced using a known genetic engineering technique.

The nucleic acid encoding the heavy chain variable region of the anti-human CD300A monoclonal antibody (i) is preferably a nucleic acid consisting of the base sequence set forth in SEQ ID NO: 33, and the nucleic acid encoding the light chain variable region of the anti-human CD300A monoclonal antibody (i) is preferably a nucleic acid consisting of the base sequence set forth in SEQ ID NO: 34.

The nucleic acid encoding the heavy chain of the anti-human CD300A monoclonal antibody (i) is preferably a nucleic acid consisting of the base sequence set forth in SEQ ID NO: 37, and the nucleic acid encoding the light chain of the anti-human CD300A monoclonal antibody (i) is preferably a nucleic acid consisting of the base sequence set forth in SEQ ID NO: 38.

It is preferable to add a nucleic acid encoding a signal peptide to a nucleic acid encoding the anti-human CD300A monoclonal antibody (X) or the antigen-binding fragment thereof. When a signal peptide of an appropriate amino acid sequence is added, an expression level of the anti-human CD300A monoclonal antibody (X) or the antigen-binding fragment thereof in cells or the amount of the antibody or the antigen-binding fragment secreted to a culture supernatant can be increased.

<Vector>

One aspect of the present invention is a vector containing a nucleic acid encoding the anti-human CD300A monoclonal antibody (X) or the antigen-binding fragment thereof. In other words, it is a recombinant vector incorporating a nucleic acid encoding the anti-human CD300A monoclonal antibody (X) or antigen-binding fragment thereof.

The vector is not particularly limited, and examples thereof include a plasmid vector and a viral vector. The vector may be a vector that can be expressed in, for example, mammals, bacteria, insects, yeast, and fungi, a vector that can be expressed in eukaryotic cells is preferable, and a vector that can be expressed in cells derived from mammals is more preferable. Examples of the vector that can be expressed in cells derived from mammals include pUC series, pCAG, pEBMulti, pEGFP-C1, pEF-BOS, pTRE-Myc, pMSCVpuro, and pCEP4, and pUC series is preferable.

A method for producing a vector is not particularly limited, and the vector can be produced by a known genetic recombination technique.

In addition to the nucleic acid encoding the anti-human CD300A monoclonal antibody (X) or the antigen-binding fragment thereof, the vector may contain a regulatory control sequence capable of controlling replication and expression in a host and/or secretion from the host. Examples of the regulatory control sequence include a promoter sequence such as a CMV promoter.

<Transformant>

One aspect of the present invention is a transformant containing a vector containing a nucleic acid encoding the anti-human CD300A monoclonal antibody (X) or the antigen-binding fragment thereof. The anti-human CD300A monoclonal antibody (X) or the antigen-binding fragment thereof can be obtained from a transformant, or a culture supernatant thereof, for example.

The transformant can be obtained by introducing the above-described recombinant vector into a host. Examples of the host include cultured cells such as E. coli, yeast, plant cells, insect cells, and animal cells, insect organisms such as silkworms, and plants such as tobacco, and animal cells are preferable.

Examples of the animal cells include mammalian cells such as NS0, Sp2/O, CHO, COS, HEK, fibroblasts, and myeloma cells, and CHO is preferable.

The introduction (transformation) of the recombinant vector into the host can be performed using a known method. Examples of such a method include a competent cell method using a cell body treated with calcium and an electroporation method. In addition, a method of infecting a host with a phage vector or a viral vector, for example, in addition to a plasmid vector, to transform the host may be used.

One aspect of the present invention is the use of the anti-human CD300A monoclonal antibody (i) or the antigen-binding fragment thereof for the treatment or prevention of a disease caused by a function of human CD300A.

One aspect of the present invention is the use of the anti-human CD300A monoclonal antibody (i) or the antigen-binding fragment thereof for the treatment or prevention of a disease associated with a function of human CD300A.

One aspect of the present invention is an anti-human CD300A monoclonal antibody (i) or an antigen-binding fragment thereof for use as a therapeutic or prophylactic agent for a disease caused by a function of human CD300A.

One aspect of the present invention is an anti-human CD300A monoclonal antibody (i) or an antigen-binding fragment thereof for use as a therapeutic or prophylactic agent for a disease associated with a function of human CD300A.

One aspect of the present invention is the use of an anti-human CD300A monoclonal antibody (i) or an antigen-binding fragment thereof in the manufacture of a therapeutic or prophylactic agent for a disease caused by a function of human CD300A.

One aspect of the present invention is the use of an anti-human CD300A monoclonal antibody (i) or an antigen-binding fragment thereof in the manufacture of a therapeutic or prophylactic agent for a disease associated with a function of human CD300A.

One aspect of the present invention is a method for treating or preventing a disease caused by a function of human CD300A, the method including administering an anti-human CD300A monoclonal antibody (i) or an antigen-binding fragment thereof to a subject.

One aspect of the present invention is a method for treating or preventing a disease associated with a function of human CD300A, the method including administering an anti-human CD300A monoclonal antibody (i) or an antigen-binding fragment thereof to a subject.

One aspect of the present invention is the use of an anti-human CD300A monoclonal antibody (ii) or an anti-human CD300A monoclonal antibody (iii), or an antigen-binding fragment thereof, for determining whether CD300A is of an arginine-type or a glutamine-type.

One aspect of the present invention is an anti-human CD300A monoclonal antibody (ii) or an anti-human CD300A monoclonal antibody (iii), or an antigen-binding fragment thereof, for use as a diagnostic agent for determining whether CD300A is of an arginine-type or a glutamine-type.

One aspect of the present invention is the use of an anti-human CD300A monoclonal antibody (ii) or an anti-human CD300A monoclonal antibody (iii), or an antigen-binding fragment thereof, in the manufacture of a diagnostic agent for determining whether CD300A is of an arginine-type or a glutamine-type.

One aspect of the present invention is a method for determining whether CD300A is of an arginine-type or a glutamine-type, the method including using an anti-human CD300A monoclonal antibody (ii) or an anti-human CD300A monoclonal antibody (iii), or an antigen-binding fragment thereof.

Examples

Next, the present invention will be described in more detail with reference to Examples, but the present invention is not limited thereto.

All experiments were performed in accordance with the guidelines of the Animal Ethics Committee of the Center for Experimental Animal Resources, University of Tsukuba. The mice were housed under specific pathogen-free conditions at the Laboratory Animal Resource Center of the University of Tsukuba.

(Production of BW cells stably expressing IRES-GFP-tagged human CD300A)

The cDNA of human CD300A$^{Arg111}$ or human CD300A$^{Gln111}$ tagged with IRES-GFP was subcloned into the pcDNA3.4 vector to generate pcDNA3.4 vectors for expression of IRES-GFP-tagged human CD300A. This was transfected into the mouse lymphoblast BW5147 cell line (hereinafter, also referred to as BW5147 cells or BW cells) to obtain cell lines stably expressing human CD300A$^{Arg111}$-IRES-GFP or human CD300A$^{Gln111}$-IRES-GFP. The cell line stably expressing human CD300A$^{Arg111}$-IRES-GFP was designated as "BW/huCD300A-R" (also referred to as "huCD300A-R/BW"), and the cell line stably expressing human CD300A$^{Gln111}$-IRES-GFP was designated as "BW/huCD300A-Q" (also referred to as "huCD300A-Q/BW"). Similarly, the cell line stably expressing human CD300C-IRES-GFP was obtained and designated as "BW/huCD300C" (also referred to as "huCD300C/BW"). In addition, untransfected BW cells were designated as "BW".

[Production of anti-human CD300A monoclonal antibody]

<Method>

(Production of immunogen)

The extracellular portion of human CD300A$^{Arg111}$ or human CD300A$^{Gln111}$ (amino acids at positions 18 to 178 of the full-length 299-amino-acid human CD300A) was fused at the C-terminus to the human IgG Fc region and subcloned into the vector pcDNA3.4 to generate the chimeric proteins (huCD300A$^{Arg111}$-Fc and huCD300A$^{Gln111}$-Fc).

(Immunization of mouse and hybridoma culture 1)

Wild-type (Balb/c) mice were immunized by intraperitoneal injection with an emulsion mixture of huCD300A$^{Arg111}$-Fc (40 µg) and complete Freund's adjuvant (manufactured by Sigma-Aldrich). At 2, 4, and 6 weeks after the immunization, the wild-type mice were immunized again by intraperitoneal injection with an emulsion mixture of huCD300A$^{Arg111}$-Fc (40 µg) and complete Freund's adjuvant (manufactured by Sigma-Aldrich). 3 days after the final immunization, spleen cells were collected and fused with SP2/O myeloma cells to obtain hybridomas.
The hybridomas were cultured overnight in HY medium C (NO. #03803, manufactured by StemCell Technologies Inc.). Thereafter, the cells were cultured in HAT medium (NO. H0137-10VL, manufactured by Sigma) and cloned. Medium was changed to medium E (manufactured by StemCell Technologies Inc., Vancouver, Canada) to allow the hybridomas to produce antibodies.

(Immunization of mouse and hybridoma culture 2)

Wild-type (Balb/c) mice were immunized by intraperitoneal injection with an emulsion mixture of huCD300A$^{Gln111}$-Fc (30 µg) and complete Freund's adjuvant (manufactured by Sigma-Aldrich). At 3, 6, and 9 weeks after the immunization, the wild-type mice were immunized again by intraperitoneal injection with an emulsion mixture of huCD300A$^{Gln111}$-Fc (30 µg) and complete Freund's adjuvant (manufactured by Sigma-Aldrich). 14 days after the final immunization, spleen cells were collected and fused with SP2/O myeloma cells to obtain hybridomas. The hybridomas were cultured by the method described in "Immunization of mouse and hybridoma culture 1".

(Immunization of mouse and hybridoma culture 3)

Wild-type (Balb/c) mice were immunized by subcutaneous injection with an emulsion mixture of huCD300A$^{Arg111}$-Fc (25 µg) and complete Freund's adjuvant (manufactured by Sigma-Aldrich). One week after, wild-type mice were immunized again by subcutaneous injection of an emulsion mixture of huCD300A$^{Arg111}$-Fc (12.5µg) and huCD300A$^{Gln111}$-Fc (12.5µg) and incomplete Freund's adjuvant (manufactured by Sigma-Aldrich). 3 days after the final immunization, draining lymph node cells were collected and fused with SP2/O myeloma cells to obtain hybridomas.
The hybridomas were cultured overnight in HY medium C (NO. #03803, manufactured by StemCell Technologies Inc.). Thereafter, the cells were cultured and cloned in HAT medium (#03804, manufactured by StemCell Technologies Inc.) in 96-well plates. Medium E (#03805, manufactured by StemCell Technologies Inc., Vancouver, Canada) was added, and the cells were cultured for 4 days to allow the hybridomas to produce antibodies.

(Screening of hybridomas)

It was analyzed by flow cytometry whether the culture supernatant of each hybridoma clone bound to "BW/huCD300A-R", "BW/huCD300A-Q", or "BW/huCD300C".

(Purification of monoclonal antibody)

After washing the hybridomas of the selected clones with PBS, the hybridomas were injected into the peritoneal cavity of nude mice primed with pristane (BALB/cAJcl-nu/nu mice purchased from CLEA Japan, Inc.). After about 10 to 14 days, ascites was collected from the mice and purified using Hitrap™ Protein A or G column (manufactured by Cytiva) to obtain purified monoclonal antibodies.

(Evaluation of binding properties of antibodies to human CD300A and human CD300C)

0.5 µg of purified monoclonal antibody was added to $1 \times 10^5$ cells of BW/huCD300A-R, BW/huCD300A-Q, BW/huCD300C, and/or BW, respectively, and incubated on ice for 30 minutes. Anti-mouse IgG2b-APC (RMG2b-1, manufactured by Abcam Limited) or Anti-mouse IgG2a-APC (RMG2a-62, manufactured by Abcam Limited) was added as a secondary antibody, and the mixture was incubated on ice for 30 minutes. Thereafter, the cells were washed with ice-cold FACS buffer and analyzed using a Fortessa flow cytometer (manufactured by Becton Dickinson). FlowJo software (Tree Star) was used for data analysis. Note that a mouse IgG isotype control antibody (MPC-11, manufactured by BioLegend) was used as a control antibody.

(Analysis of inhibition (neutralization function) of binding between human CD300A protein and dead cells by antibody)

Whether the purified monoclonal antibody neutralizes the binding of huCD300A$^{Arg111}$-Fc or huCD300A$^{Gln111}$-Fc to PS, which is a ligand of human CD300A, was analyzed by the following method.

Thymocytes were isolated from C57BL/6 mice (purchased from CLEA Japan, Inc.) and incubated for 12 hours in DMEM containing 10% FBS and 1 $\mu$M dexamethasone (manufactured by Sigma-Aldrich) to induce apoptosis. The induced apoptotic cells were washed with Annexin V binding buffer, and then, huCD300A$^{Arg111}$-Fc or huCD300A$^{Gln111}$-Fc, and the purified monoclonal antibody were added and incubated at room temperature for 30 minutes. Thereafter, the cells were washed, Annexin V-APC, anti-human IgG Fc antibody (M1310G05) (NO. 410708, manufactured by BioLegend), and propidium iodide (PI) were added, the cells were stained on ice for 30 minutes, and then analysis was performed by flow cytometry. Mouse IgG2b (NO. ab18469, manufactured by Abcam Limited) was used as an isotype control for the purified monoclonal antibody.

<Results>

Clones of hybridomas obtained by the method described in "Immunization of mouse and hybridoma culture 3" included: clones that produced an antibody (Antibody NO. 1) which reacted with both "BW/huCD300A-R" and "BW/huCD300A-Q" and did not react with "BW/huCD300C"; and clones that produced an antibody (Antibody NO. 2) which reacted with "BW/huCD300A-R" andreacted with neither "BW/huCD300A-Q" and "BW/huCD300C.

Among the hybridoma clones obtained by the method described in "Immunization of mouse and hybridoma culture 2", there were clones that produced an antibody (Antibody NO. 3) which reacted with "BW/huCD300A-Q" and reacted with neither "BW/huCD300A-R" nor "BW/huCD300C."

Among the hybridoma clones obtained by the method described in "Immunization of mouse and hybridoma culture 1", there were clones that produced an antibody (Antibody NO. 4) which reacted with "BW/huCD300A-R," "BW/huCD300A-Q," and "BW/huCD300C"; and clones that produced an antibody (Antibody NO. 5) which reacted with "BW/huCD300A-R" and "BW/huCD300A-Q" and did not react with "BW/huCD300C."

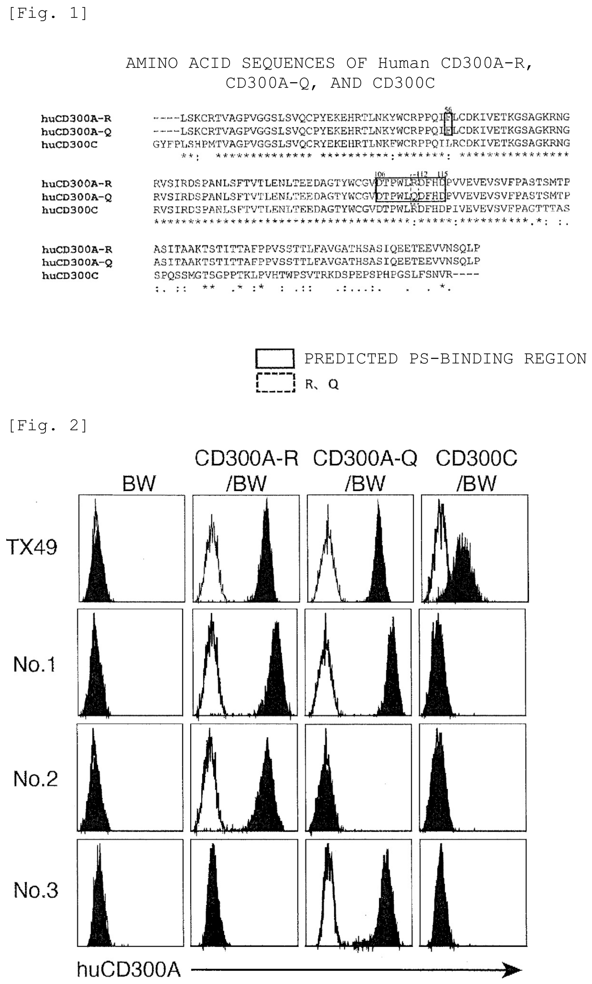

Fig. 1 shows a comparison of the amino acid sequences near the ligand-binding sites of human CD300A-R, human CD300A-Q, and human CD300C.

Fig. 2 shows the results of the evaluation of the binding properties of Antibody NO. 1, Antibody NO. 2, and Antibody NO. 3 to human CD300A and human CD300C. In addition, TX49 (anti-human CD300A antibody described in Patent Literature 1) was also evaluated as a representative existing anti-human CD300A antibody. Representative data from two or three independent experiments are shown. The vertical axis of the histogram indicates the number of cells, and the horizontal axis indicates huCD300A.

It was revealed that TX49 binds to both human CD300A-R and human CD300A-Q, and also binds to human CD300C.

It was revealed that Antibody NO. 1 binds to human CD300A-R and human CD300A-Q, but does not bind to human CD300C.

It was revealed that Antibody NO. 2 binds to human CD300A-R, but does not bind to human CD300A-Q or human CD300C.

It was revealed that Antibody NO. 3 binds to human CD300A-Q, but does not bind to human CD300A-R or human CD300C.

Although not shown in the drawings, similar experiments revealed that Antibody NO. 4 binds to human CD300A-R, human CD300A-Q, and human CD300C, and that Antibody NO. 5 binds to human CD300A-R and human CD300A-Q but does not bind to human CD300C.

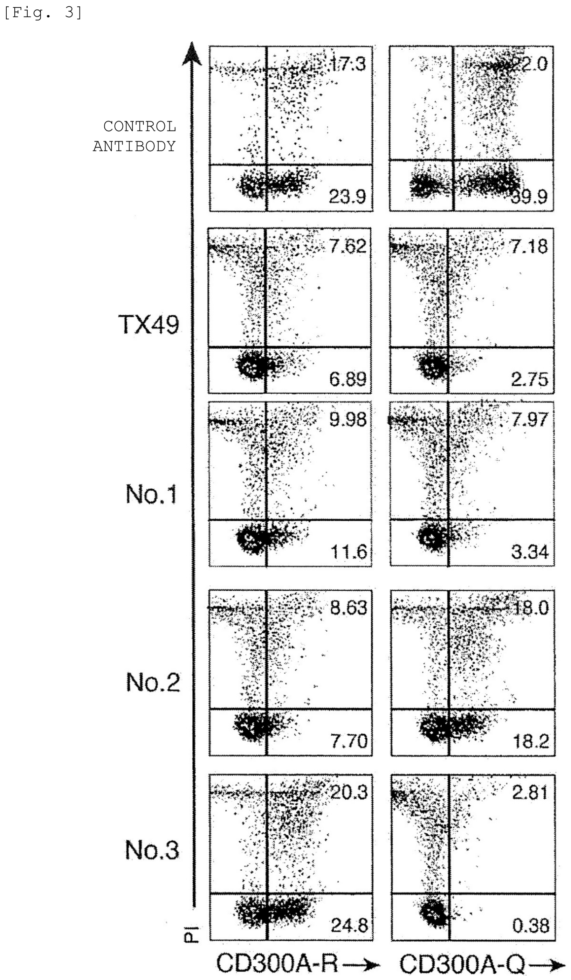

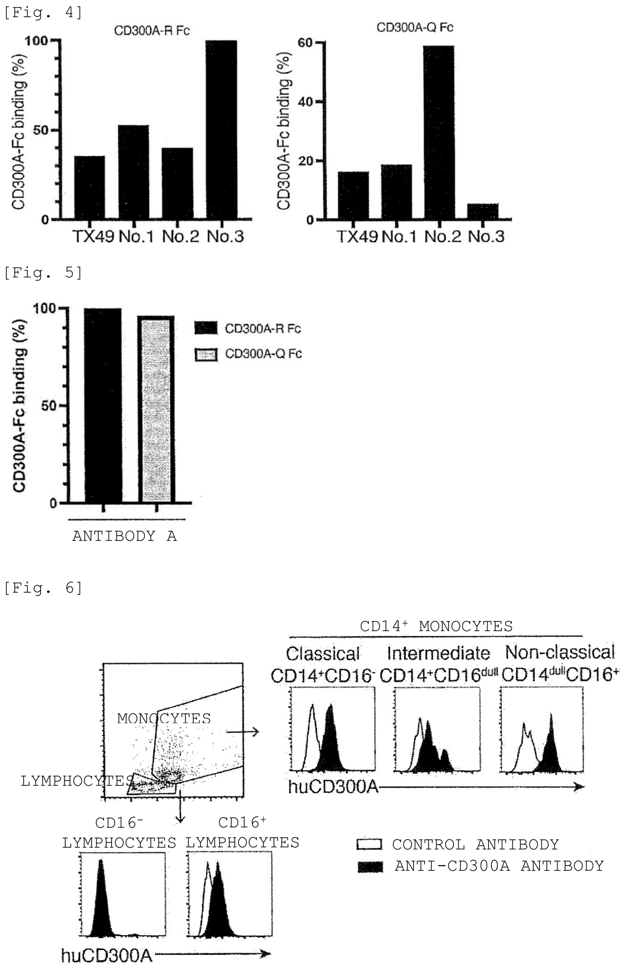

Figs. 3 and 4 show the results of the analysis of the neutralizing function of Antibody NO. 1, Antibody NO. 2, and Antibody NO. 3. In addition, evaluations were also conducted for TX49 (Figs. 3 and 4), a representative existing anti-human CD300A antibody, and Antibody A (Fig. 5).

TX49 inhibited both the binding of human CD300A-R to apoptotic cells and the binding of human CD300A-Q to apoptotic cells. It was revealed that TX49 has the function to neutralize the binding of human CD300A-R to PS as well as the binding of human CD300A-Q to PS.

Antibody NO. 1 inhibited both the binding of human CD300A-R to apoptotic cells and the binding of human CD300A-Q to apoptotic cells. It was revealed that Antibody NO. 1 has the function to neutralize the binding of human CD300A-R to PS as well as the binding of human CD300A-Q to PS.

Antibody NO. 2 inhibited the binding of human CD300A-R to apoptotic cells, but showed little inhibition of the binding of human CD300A-Q to apoptotic cells. It was revealed that Antibody NO. 2 has the function to neutralize the binding of human CD300A-R to PS, but does not have the function to neutralize the binding of human CD300A-Q to PS.

Antibody NO. 3 almost completely inhibited the binding of human CD300A-Q to apoptotic cells, but showed little inhibition of the binding of human CD300A-R to apoptotic cells. It was revealed that Antibody NO. 3 has the function to neutralize the binding of human CD300A-Q to PS, but does not have the function to neutralize the binding of human CD300A-R to PS.

Although not shown in the drawings, similar experiments revealed that Antibody NO. 4 did not inhibit either the binding of human CD300A-Q to apoptotic cells or the binding of human CD300A-R to apoptotic cells. Independent experiments were performed twice and the same results were obtained in both. It was revealed that Antibody NO. 4 does not have a function to neutralize the binding of human CD300A-Q to PS, nor a function to neutralize the binding of human CD300A-R to PS.

In addition, although not shown in the drawings, similar experiments revealed that Antibody NO. 5 did not inhibit either the binding of human CD300A-Q to apoptotic cells or the binding of human CD300A-R to apoptotic cells. Independent experiments were performed twice and the same results were obtained in both. It was revealed that Antibody NO. 5 does not have a function to neutralize the binding of human CD300A-Q to PS, nor a function to neutralize the binding of human CD300A-R to PS.

Antibody A did not inhibit both the binding of human CD300A-Q to apoptotic cells and the binding of human CD300A-R to apoptotic cells (Fig. 5). It was revealed that Antibody A does not have a function to neutralize the binding of human CD300A-Q to PS, nor a function to neutralize the binding of human CD300A-R to PS.

[Expression of CD300A in human peripheral blood cells]

<Method>

Blood was collected from healthy volunteers (amino acid at position 111 of CD300A is R), and peripheral blood mononuclear cells (PBMCs) were collected using Lymphoprep™ (manufactured by STEMCELL Technologies Inc.) in accordance with the manufacturer's instructions. PBMCs were stained with Antibody NO. 1 as a primary antibody after Fc receptor blocking, and then stained with anti-mouse IgG2b-APC antibody (NO. ab130792, manufactured by BioLegend) as a secondary antibody and antibodies against cell surface markers.

As antibodies against cell surface markers, anti-human CD14-FITC antibody (NO. 555397, manufactured by BD Biosciences) and anti-human CD16-PE antibody (NO. 555407, manufactured by BD Biosciences) were used. The clone names are indicated in parentheses.

Gating was performed as shown below.

Monocyte

Classical: CD14$^+$CD16$^-$
Intermediate: CD14$^+$CD16$^{dull}$
Non-Classical: CD14$^{dull}$CD16$^+$

<Results>

The results are shown in Fig. 6.

It was revealed that human CD300A-R is barely expressed on CD16$^-$ lymphocytes, whereas expression is observed on CD16$^+$ lymphocytes. In addition, it was revealed that human CD300A-R is expressed on monocytes in general.

(Binding of antibody to Macaca fascicularis peripheral blood cells)

<Method>

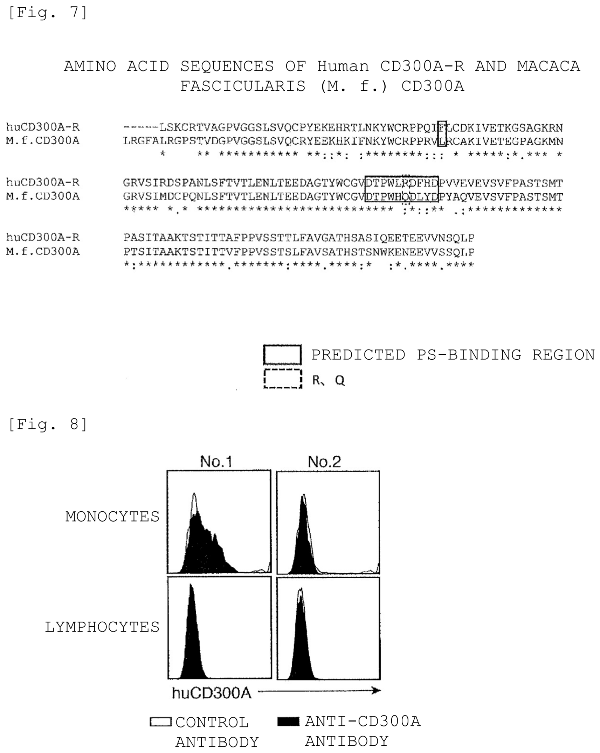

Fig. 7 shows the amino acid sequence near the ligand-binding site of human CD300A-R and the amino acid sequence of the corresponding site of *Macaca fascicularis.* In Fig. 7, the predicted PS-binding region is indicated by a solid line, and the arginine (R) or glutamine (Q) at position 111 is indicated by a dashed line.

Peripheral blood was collected with heparin from Macaca fascicularis, and peripheral blood mononuclear cells (PBMCs) were isolated by density gradient separation using Lympholyte-Mammal (manufactured by Cedarlane Corporation). After Fc receptor blocking of PBMCs, the cells were stained with the purified monoclonal antibody and analyzed by flow cytometry. Mouse IgG2b (NO. ab18469, manufactured by Abcam Limited) was used as a control antibody.

<Results>

The results are shown in Fig. 8. The upper two correspond to results using monocytes, and the lower two correspond to results using lymphocytes.

Macaca fascicularis CD300A was not expressed on lymphocytes but was expressed on monocytes. Antibody NO. 1 reacted with Macaca fascicularis CD300A, whereas Antibody NO. 2 did not react with Macaca fascicularis CD300A.

In the development of antibody therapeutics, it is important that the antibody reacts with an appropriate animal species expected to have extrapolatability to humans so that preclinical safety tests and other studies can be performed using the animal species. It has been revealed that Antibody NO. 1 has a characteristic useful for development as an antibody therapeutic in that preclinical safety tests and other studies can be performed using Macaca fascicularis.

(Sequence determination of monoclonal antibody)

<Method>

The genes of the variable regions of the heavy chains and light chains of Antibody NO. 1, Antibody NO. 2, and Antibody NO. 3 were cloned, and the base sequences were determined by PCR method. Details of the method are as described in Thomas Tiller et. al., Journal of immunological methods, 350, 2009, 183-193.

Amino acid sequences were estimated from the base sequences, and the complementarity determining regions (CDRs) of the variable regions of the heavy chains and light chains of Antibody NO. 1, Antibody NO. 2, and Antibody NO. 3 were determined using abYsis according to the Kabat method (Kabat et al. 1991, Sequences of Proteins of Immunological Interests, Fifthe Edition, NIH Publication No. 91-3242, U.S. Department of Health and Human Services).

<Results>

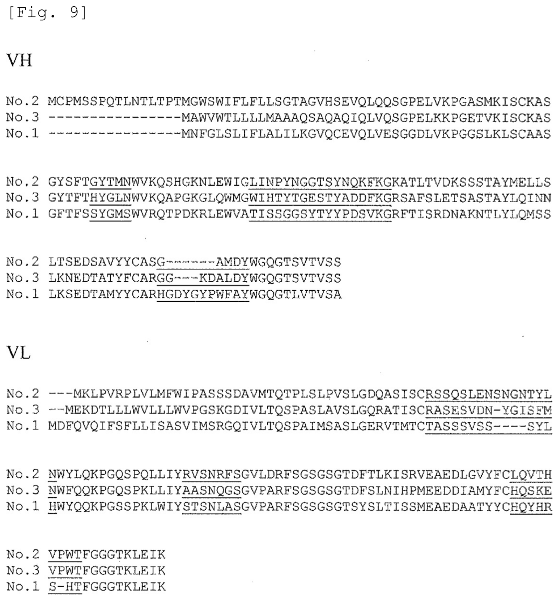

Fig. 9 shows the amino acid sequences of the variable regions and CDR1 to CDR3 of the heavy chains and light chains of Antibody NO. 1, Antibody NO. 2, and Antibody NO. 3. The CDR1 to CDR3 in the variable regions of the heavy chain and light chain were indicated with underlining.

[Production of humanized antibody]

<Production of chimeric antibody>

To construct the expression vector for mouse-human chimeric NO. 1 IgG1/kappa antibody, the gene encoding the mouse Antibody NO. 1 VH was designed as an exon containing a splice donor signal downstream of the coding region and restriction enzyme sites at the 5' and 3' ends Similarly, the gene encoding the mouse antibody NO. 1 VL was designed as an exon containing a splice donor signal downstream of the coding region and restriction enzyme sites at the 5' and 3' ends. In order to produce chimeric NO. 1 IgG1/kappa antibody (Ch NO. 1), the synthesized NO. 1 VH and VL genes were inserted into mammalian expression vectors containing the human γ-1 and κ constant regions using restriction enzymes. The resulting expression vector is designated as pCh NO. 1. The γ-1 heavy chain gene of pCh NO. 1 contains amino acid substitutions from leucine to alanine at positions 234 and 235 (according to Kabat numbering) (L234A/L235A), and it is known that the effector function of the IgG antibody disappears.

<Design of VH and VL genes of humanized antibodies>

Based on Antibody NO. 1, the amino acid sequences of VH (heavy chain variable region) and VL (light chain variable region) of the humanized antibody were designed as follows. First, a three-dimensional molecular model of Antibody NO. 1 variable region was constructed. Using the molecular model, framework amino acid residues important for the formation of the three-dimensional structure of CDRs were identified.

(Examination of VH of humanized antibody)

A human VH sequence homologous to the framework of Antibody NO. 1 VH was searched in the GenBank database, and the VH sequence encoded by human U96282 cDNA (U96282 VH) was selected as the acceptor for humanization.

The HCDR sequences of Antibody NO. 1 were grafted onto the corresponding positions of U96282 VH and this was defined as Hu NO. 1 VH1.

Because it was considered important for appropriate formation of the antigen-binding site, the amino acid residue at position 44 of Hu NO. 1 VH1 was substituted from G (glycine), which is the amino acid residue in U96282 VH, to R (arginine), which is the corresponding residue in Antibody NO. 1 VH. The obtained VH was defined as Hu NO. 1 VH2.

Furthermore, in order to more appropriately form the antigen-binding site, a Hu NO. 1 VH2 mutant was designed by introducing the following mutations into Hu NO. 1 VH2.

The amino acid residue at position 19 of Hu NO. 1 VH2 was substituted from R (arginine), which is the amino acid residue in U96282 VH, to K (lysine), which is the corresponding residue in Antibody NO. 1 VH. The obtained VH was defined as Hu NO. 1 VH2 R19K.

The amino acid residue at position 42 of Hu NO. 1 VH2 was substituted from G (glycine), which is the amino acid residue in U96282 VH, to D (aspartic acid), which is the corresponding residue in Antibody NO. 1 VH. The obtained VH was designated as Hu NO. 1 VH2 G42D (also referred to as Hu NO. 1 VH3).

The amino acid residue at position 89 of Hu NO. 1 VH2 was substituted from V (valine), which is the amino acid residue in U96282 VH, to M (methionine), which is the corresponding residue in Antibody NO. 1 VH. The obtained VH was defined as Hu NO. 1 VH2 V89M.

The amino acid residue at position 10 of Hu NO. 1 VH2 was substituted from G (glycine), which is the amino acid residue in U96282 VH, to D (aspartic acid), which is the corresponding residue in Antibody NO. 1 VH. The obtained VH was defined as Hu NO. 1 VH2 G10D.

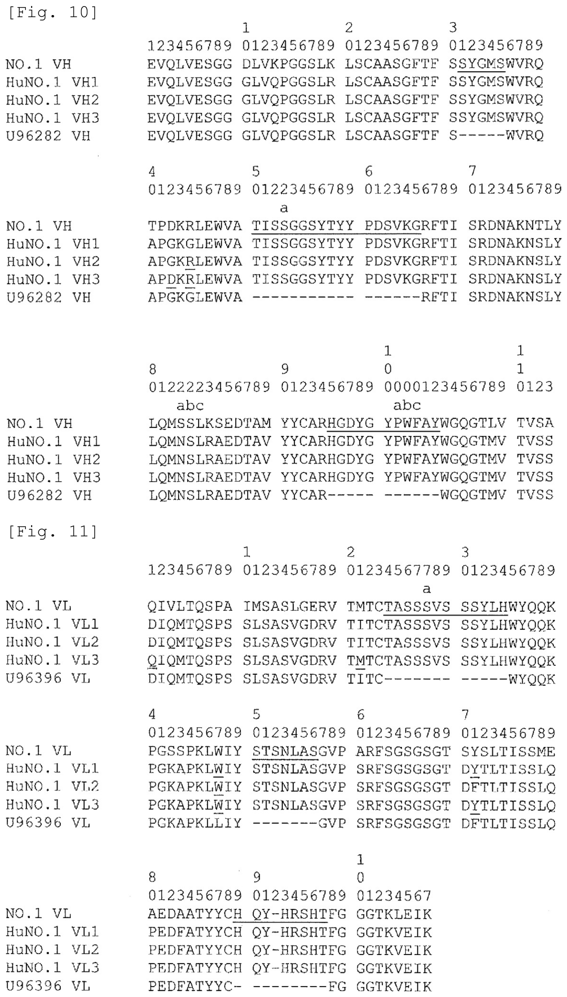

Fig. 10 shows the amino acid sequences of Antibody NO. 1 VH (SEQ ID NO: 7), Hu NO. 1 VH1, Hu NO. 1 VH2, Hu NO. 1 VH3 (SEQ ID NO: 31), and U96282 VH.

(Examination of VL of humanized antibody)

A human VH sequence homologous to the framework of Antibody NO. 1 VL was searched in the GenBank database, and the VL sequence encoded by human U96396 cDNA (U96396 VL) was selected as the acceptor for humanization.

The LCDR sequences of Antibody NO. 1 were grafted onto the corresponding positions of U96396 VL, and furthermore, because these amino acid residues were considered important for CDR structure formation, the amino acid residue at position 47 was substituted from L (leucine), which is the amino acid residue in U96396 VL to W (tryptophan), which is the corresponding residue in Antibody NO. 1 VL, and the amino acid residue at position 71 was substituted from F (phenylalanine), which is the amino acid residue in U96396 VL to Y (tyrosine), which is the corresponding residue in Antibody NO. 1 VL. The obtained VL was defined as Hu NO. 1 VL1.

When the steric structure of CDR was further analyzed, it was considered that the amino acid residue at position 71 might not require substitution from F (phenylalanine), which is the amino acid residue in U96396 VL to Y (tyrosine), which is the corresponding residue in Antibody NO. 1 VL, as this substitution may not affect antigen-binding affinity, and therefore, the amino acid residue at position 71 of Hu NO. 1 VL1 was substituted from Y (tyrosine) to F (phenylalanine). The obtained VH was defined as Hu NO. 1 VL2.

Furthermore, in order to more appropriately form the antigen-binding site, a Hu NO. 1 VL1 mutant was designed by introducing the following mutations into Hu NO. 1 VL1.

The amino acid residue at position 1 of Hu NO. 1 VL1 was substituted from D (aspartic acid), which is the amino acid residue in U96396 VL, to Q (glutamine), which is the corresponding residue of Antibody NO. 1 VL. The obtained VL was defined as Hu NO. 1 VL1 D1Q.

The amino acid residue at position 3 of Hu NO. 1 VL1 was substituted from Q (glutamine), which is the amino acid residue in U96396 VL, to V (valine), which is the corresponding residue of Antibody NO. 1 VL. The obtained VL was defined as Hu NO. 1 VL1 Q3V.

The amino acid residue at position 4 of Hu NO. 1 VL1 was substituted from M (methionine), which is the amino acid residue in U96396 VL, to L (leucine), which is the corresponding residue in Antibody NO. 1 VL. The obtained VL was defined as Hu NO. 1 VL1 M4L.

The amino acid residue at position 21 of Hu NO. 1 VL1 was substituted from I (isoleucine), which is the amino acid residue in U96396 VL, to M (methionine), which is the corresponding residue in Antibody NO. 1 VL. The obtained VL was defined as Hu NO. 1 VL1 I21M.

The amino acid residue at position 42 of Hu NO. 1 VL1 was substituted from K (lysine), which is the amino acid residue in U96396 VL, to S (serine), which is the corresponding residue of Antibody NO. 1 VL. The obtained VL was defined as Hu NO. 1 VL1 K42S.

The amino acid residue at position 70 of Hu NO. 1 VL1 was substituted from D (aspartic acid), which is the amino acid residue in U96396 VL, to S (serine), which is the corresponding residue of Antibody NO. 1 VL. The obtained VL was defined as Hu NO. 1 VL1 D70S.

The amino acid residue at position 1 of Hu NO. 1 VL1 was substituted from D (aspartic acid), which is the amino acid residue in U96396 VL, to Q (glutamine), which is the corresponding residue of Antibody NO. 1 VL, and furthermore, the amino acid residue at position 21 of Hu NO. 1 VL1 was substituted from I (isoleucine), which is the amino acid residue in U96396 VL, to M (methionine), which is the corresponding residue of Antibody NO. 1 VL. The obtained VL was designated Hu NO. 1 VL1 D1Q/I21M (also referred to as Hu NO. 1 VL3).

Fig. 11 shows the amino acid sequences of Antibody NO. 1 VL (SEQ ID NO: 8), Hu NO. 1 VL1, Hu NO. 1 VL2, Hu NO. 1 VL3 (SEQ ID NO: 32), and U96396 VL.

(Expression of VH and VL of humanized antibody)

The genes encoding each VH and VL were designed to include a signal peptide, splice donor signal, and restriction enzyme sites at the 5' and 3' ends. The synthesized genes were incorporated into plasmids in the combinations shown in Tables 1, 2, and 3 below to produce expression vectors.

[Table 1]

Table 1

| Plasmid | VH | VL | Expressed antibody |
|---|---|---|---|
| pChHuNO.1a | HuNO.1 VH1 | NO.1 VL | ChHuNO.1a |
| pChHuNO.1b | HuNO.1 VH2 | NO.1 VL | ChHuNO.1b |
| pChHuNO.1c | NO.1 VH | Hu NO.1 VL1 | ChHuNO.1c |
| pChHu NO.1d | NO.1 VH | Hu NO.1 VL2 | ChHuNO.1d |

[Table 2]

Table 2

| Plasmid | VH | VL | Expressed antibody |
|---|---|---|---|
| pChHuNO.1e | NO.1 VH | HuNO.1 VL1 D1Q | ChHuNO.1e |
| pChHuNO.1f | NO.1 VH | HuNO.1 VL1 Q3V | ChHuNO.1f |
| pChHuNO.1g | NO.1 VH | HuNO.1 VL1 M4L | ChHuNO.1g |
| pChHuNO.1h | NO.1 VH | HuNO.1 VL1 I21M | ChHuNO.1h |
| pChHuNO.1i | NO.1 VH | HuNO.1 VL1 K42S | ChHuNO.1i |
| pChHuNO.1j | NO.1 VH | HuNO.1 VL1 D70S | ChHuNO.1j |
| pChHuNO.1k | NO.1 VH | HuNO.1 VL1 DIQ/I21M (HuNO.1 VL3) | ChHuNO.1k |

[Table 3]

Table 3

| Plasmid | VH | VL | Expressed antibody |
|---|---|---|---|
| pHuNO.1R | HuNO.1 VH2 | HuNO.1 VL3 | HuNO.1R |
| pHuNO.1T | HuNO.1 VH2 R19K | HuNO.1 VL3 | HuNO.1T |
| pHuNO.1U | HuNO.1 VH2 G42D (HuNO.1 VH3) | HuNO.1 VL3 | HuNO.1U |
| pHuNO.1V | HuNO.1 VH2 V89M | HuNO.1 VL3 | HuNO.1V |
| pHuNO.1W | HuNO.1 VH2 G10D | HuNO.1 VL3 | HuNO.1W |

Each expression vector was transfected into human embryonic kidney cell line HEK293 using polyethyleneimine, and recombinant antibodies were transiently expressed. HEK293 cells were cultured in a 7.5% $CO_2$ incubator at 37°C using DMEM medium containing 10% FBS (manufactured by Hyclone Laboratories Inc.).

Expression of the antibody (test antibody) in the culture supernatant of transiently transfected HEK293 cells was confirmed by sandwich ELISA. The ELISA plate was coated overnight at 4°C with 100 $\mu$l/well of goat anti-human IgG Fc$\gamma$-specific polyclonal antibody (manufactured by Jackson ImmunoResearch Inc.) diluted 1/2,000 in PBS, washed with washing buffer (PBS containing 0.05% Tween 20), and then blocked with 300 $\mu$l/well of ELISA buffer (PBS containing 2% skim milk and 0.05% Tween 20). After washing with washing buffer, 100 $\mu$l/well of the test antibody appropriately diluted with an ELISA buffer was added to the ELISA plate. A humanized IgG1/kappa antibody was used as a standard. The ELISA plate was incubated at room temperature for 1 hour and washed with washing buffer, and then a bound test antibody was detected using 100 $\mu$l/well of HRP-conjugated goat anti-human $\kappa$-chain polyclonal antibodies (manufactured by Bethyl Laboratories) diluted to 1/2,000. The ELISA plate was incubated at room temperature for 0.5 hours and washed with washing buffer, 100 $\mu$l/well of ABTS substrate (manufactured by Thermo Scientific) was added to start color development, and the incubation was stopped with 100 $\mu$l/well of 2% oxalic acid. Absorbance at 405 nm was read.

[Binding affinity of humanized antibody to antigen]

The binding affinity of the antibody (test antibody) produced by the transfected cells to human CD300A was examined using ELISA and flow cytometry.

(ELISA)

In the ELISA, the wells of an ELISA plate were coated overnight with 1 $\mu$g/ml human CD300A-Fc fusion protein (the extracellular domain of the R111 allele of CD300A fused to human Fc$\gamma$1, Cat. 12449-H02H, manufactured by Sino Biological, Inc.) dissolved in PBS, washed with washing buffer, and then blocked with ELISA buffer. After washing the wells with washing buffer, the test antibody, appropriately diluted with ELISA buffer was added at 100 $\mu$l/well and incubated for 1 hour. After washing the wells with wash buffer, antibodies bound to human CD300A were detected with HRP-labeled goat anti-human $\kappa$-chain polyclonal antibodies. Color development was performed with ABTS substrate.

(Flow cytometry analysis)

ST594 cells expressing the R111 allele of human CD300A on the surface were used. ST594 cells were cultured at 37°C in a 7.5% $CO_2$ incubator using RPMI-1640 medium containing 10% FBS, 50 $\mu$M 2-mercaptoethanol, 2 mM L-glutamine, 100 U/ml penicillin, 0.1 mg/ml streptomycin, 10 mM HEPES, 1 mM sodium pyruvate, and 100 $\mu$M MEM non-essential amino acids.

In a typical experiment, the test antibody was incubated at various concentrations with about $10^5$ ST594 cells in PBS containing 0.5% BSA and 0.05% NaN3 (FACS buffer) at 4°C for 1 hour.

Next, the cells were washed with ice-cold FACS buffer and incubated at 4°C for 30 minutes with APC-labeled goat anti-human IgG antibody (SouthernBiotech, Birmingham). After washing with FACS buffer and suspending, the stained cells were analyzed by flow cytometry.

(Results)

From the analysis results by ELISA and flow cytometry obtained by comparing Ch NO. 1 with the four types shown in Table 1, it was revealed that all of ChHu NO. 1a, b, c, and d bound to human CD300A-R, but the binding affinity thereof was inferior to that of Ch NO. 1. In addition, regarding antigen-binding affinity, Hu NO. 1 VH2 was shown to be superior to Hu NO. 1 VH1, and Hu NO. 1 VL1 was shown to be superior to Hu NO. 1 VL2.

From the analysis results by ELISA and flow cytometry obtained by comparing Ch NO. 1, ChHu NO. 1c, and ChHu NO. 1e to ChHu NO. 1j, and the analysis results by ELISA and flow cytometry obtained by comparing Ch NO. 1, ChHu NO. 1c, and ChHu NO. 1e to ChHu NO. 1k, it was revealed that all seven Hu NO. 1 VL1 variants (ChHu NO. 1e to ChHu NO. 1k) shown in Table 2 bind to human CD300A-R.

Among the top six Hu NO. 1 VL1 variants shown in Table 2 (ChHu NO. 1e to ChHu NO. 1j), ChHu NO. 1e (NO. 1 VH1 + Hu NO. 1 VL1 D1Q) and ChHu NO. 1h (NO. 1 VH + Hu NO. 1 VL1 I21M) exhibited higher binding affinity to CD300A-R compared to the other four variants.

Furthermore, ChHu NO. 1k (NO. 1 VH + Hu NO. 1 VL1 D1Q/I21M) exhibited higher binding affinity to CD300A-R than both ChHu NO. 1e (NO. 1 VH + Hu NO. 1 VL1 D1Q) and ChHu NO. 1h (NO. 1 VH + Hu NO. 1 VL1 I21M). Therefore, Hu NO. 1 VL1 D1Q/I21M (Hu NO. 1 VL3) was selected as the VL of the humanized antibody.

The analysis results obtained by ELISA comparing Ch NO. 1, ChHu NO. 1k, and Hu NO. 1R revealed that Hu NO. 1R is inferior in binding affinity to human CD300A-R to Ch NO. 1 and ChHu NO. 1k.

The analysis results obtained by ELISA comparing Ch NO. 1 and Hu NO. 1R to Hu NO. 1V, flow cytometry analysis using CD300A-R comparing Ch NO. 1 with Hu NO. 1R through Hu NO. 1V, and flow cytometry analysis using CD300A-Q comparing Ch NO. 1 with Hu NO. 1R, Hu NO. 1U, and Hu NO. 1W revealed that all five Hu NO. 1 VH2 variants (Hu NO. 1R to Hu NO. 1W) shown in Table 3 bind to both human CD300A-R and human CD300A-Q.

Among the five variants shown in Table 3, Hu NO. 1U exhibited binding affinity to both CD300A-R and CD300A-Q that was closest to Ch NO. 1 compared to the other four variants.

These results revealed that among the antibodies produced, Hu NO. 1U is the most excellent as Humanized NO. 1 antibody.

[Establishment of cell line stably producing antibody]

In order to obtain cell lines stably producing Ch NO. 1 or Hu NO. 1U, the expression vectors pCh NO. 1 or pHu NO. 1U were introduced into the chromosomes of Chinese hamster ovary cell line CHO-K1 (obtained from ATCC) by the following method, respectively.

CHO-K1 cells were cultured in a 7.5% $CO_2$ incubator at 37°C using SFM4CHO medium (manufactured by Hyclone Laboratories Inc.). Transfection into CHO-K1 was performed by electroporation. Prior to the transfection, each expression vector was linearized using FspI. About $2.5 \times 10^6$ cells were transfected with 20 μg of a linearized plasmid and suspended in SFM4CHO medium, the cells were appropriately diluted, and then the cells were plated in a plurality of 96-well plates. After 48 hours, 10 μg/ml of puromycin was added and stable transfectants were separated. About 10 days after the start of the selection, antibody production of the culture supernatant of the transfectant placed in the 96-well plate was measured by sandwich ELISA by the method described above. CHO-K1 stable transfectants producing Ch NO. 1 or Hu NO. 1U at high levels were selected and subjected to the next culture.

The CHO-K1 stable transfectants were cultured in SFM4CHO medium in roller bottles and further cultured until cell viability was less than 50%. After centrifugation and filtration, the culture supernatant was loaded onto Protein A Sepharose column (HiTrap MabSelect SuRe, manufactured by GE Healthcare Technologies, Inc.). After washing the column with PBS, antibodies were eluted with a 0.1 M glycine-HCl buffer (pH 3.0) containing 0.1 M NaCl. After neutralization with 1 M Tris-HCl (pH 8.0), the buffer of eluted antibodies was exchanged into PBS by dialysis.

The antibody concentrations determined by measuring absorbance at 280 nm (1.4OD = 1 mg/ml) were 4.3 mg/ml for Ch NO. 1, 3.0 mg/ml for the first Hu NO. 1U clone, and 3.4 mg/ml for the second Hu NO. 1U clone. The yields were 52 mg for Ch NO. 1 from 500 ml of culture supernatant, 30 mg for the first Hu NO. 1U clone from 1,000 ml, and 36 mg for the second Hu NO. 1U clone from 1,000 ml.

The characteristics of the purified Ch NO. 1 and Hu NO. 1U were examined by SDS-PAGE according to a standard procedure. As a result of analysis under reducing conditions, it was found that each of these antibodies was composed of a heavy chain having a molecular weight of about 50 kDa and a light chain having a molecular weight of about 25 kDa. In addition, it was found that the purity of each antibody was 95% or more.

The sequence correctness of the heavy chain and the light chain produced by two clones of CHO-K1/pHu NO. 1U was confirmed by cDNA sequencing.

The base sequences of the coding regions of the heavy chain and the light chain of Hu NO. 1U completely matched the corresponding sequences of pHu NO. 1U in both of the two clones.

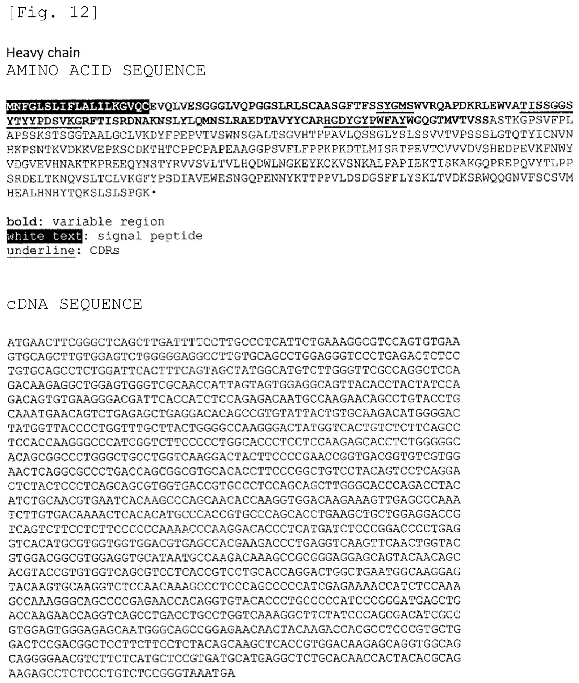

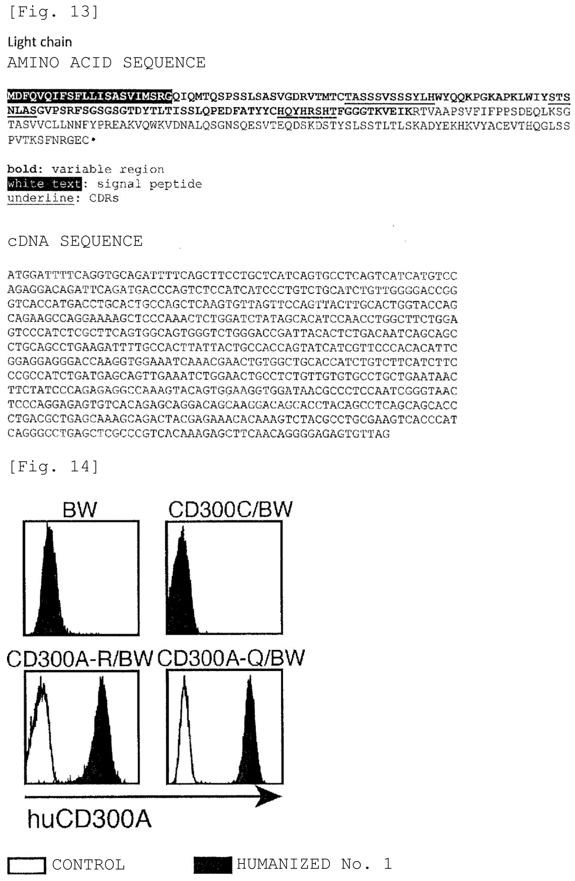

The cDNA sequences of the heavy chain and light chain encoded by pHu NO. 1U are shown in Figs. 12 and 13, respectively, together with the estimated amino acid sequences. The CDR1 to CDR3 in the variable regions of the heavy chain and light chain were indicated with underlining. The CDR1 to CDR3 were determined using abYsis according to the Kabat method (Kabat et al. 1991, Sequences of Proteins of Immunological Interests, Fifthe Edition, NIH Publication No. 91-3242, U.S. Department of Health and Human Services).

(Evaluation of binding properties of purified Hu NO. 1U to human CD300A and human CD300C)

<Method>

0.5 μg of biotinylated Hu NO. 1U was added to $1 \times 10^5$ cells each of BW/huCD300A-R, BW/huCD300A-Q, BW/huCD300C, and BW, and incubated on ice for 30 minutes. Thereafter, the cells were washed with ice-cold FACS buffer, and SA-APC was added as a secondary antibody, and then, the cells were incubated on ice for 30 minutes. Thereafter, the cells were washed with ice-cold FACS buffer and analyzed using a Fortessa flow cytometer (manufactured by Becton Dickinson). FlowJo software (Tree Star) was used for data analysis. Note that the case of staining with only the secondary antibody without using the primary antibody was used as a control.

<Results>

The results are shown in Fig. 14. Representative data from two or three independent experiments are shown. The vertical axis of the histogram indicates the number of cells, and the horizontal axis indicates huCD300A.
Hu NO. 1U (Humanized NO. 1) was revealed to bind to human CD300A-R and human CD300A-Q, but not to human CD300C, similarly to Antibody NO. 1.

(Determination of $K_D$ value of purified Hu NO. 1U)

The $K_D$ value of Hu NO. 1U against human CD300A-R was measured by the biolayer interferometry (BLI) method.
The $K_D$ value of Hu NO. 1U against human CD300A-R was $2.2 \times 10^{-8}$ M.

(Comparison of binding properties between Hu NO. 1U and Antibody NO. 1)

<Method>

The binding properties of 0.5 μg each of Antibody NO. 1 and Hu NO. 1U to human CD300A-R and human CD300A-Q were evaluated in the same manner as described above.

<Results>

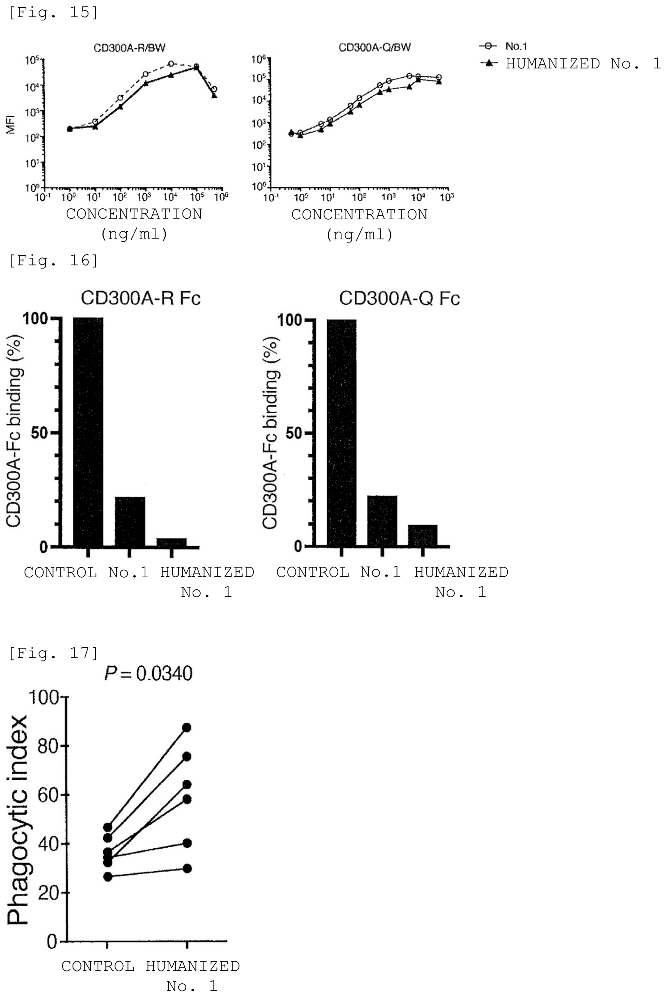

The results are shown in Fig. 15.
Hu NO. 1U (Humanized NO. 1) bound to BW/huCD300A-R and BW/huCD300A-Q. Although the binding affinity was inferior to that of Antibody NO. 1, it was still sufficiently high.

(Neutralization function of Hu NO. 1U)

<Method>

The neutralization function of Hu NO. 1U was analyzed by the above-described method. In addition, Antibody NO. 1 was also evaluated.

<Results>

The results are shown in Fig. 16.
It was revealed that Hu NO. 1U has the function to neutralize the binding of human CD300A-R to PS and the binding of human CD300A-Q to PS, similarly to Antibody NO. 1. The neutralization function of Hu NO. 1U tended to be stronger than that of Antibody NO. 1.

(Enhancement of efferocytosis of human peripheral blood monocytes by Hu NO. 1U)

<Method>

Human peripheral blood monocytes were purified from peripheral blood collected from healthy volunteers (amino acid at position 111 of CD300A is R) using the EasySep™ Human Monocyte Isolation Kit (manufactured by STEMCELL Technologies). The human peripheral blood monocytes were cultured in RPMI 1640 medium containing 10% FBS and 10 ng/mL human GM-CSF (manufactured by BioLegend, Inc.) for 5 days to differentiate into macrophages. The above-described apoptotic thymocytes were stained with 100 ng/mL pHrodo-SE (manufactured by Invitrogen) at room temperature for 30 minutes. To macrophages pre-incubated with Hu NO. 1U for 30 minutes, stained apoptotic thymocytes were added at a ratio of 10:1 (the number of apoptotic thymocytes:the number of macrophages) and incubated at 37°C for 2 hours.

Phagocytosis by macrophages was analyzed by detecting pHrodo fluorescence using Fluoview FV10i confocal microscope. Digital images of randomly selected fields under a phase-contrast microscope were captured, the number of macrophages and CD11b$^+$ cells taken up into the macrophages (100 cells or more) in each field were counted, and the phagocytic index was calculated according to the following formula.

Phagocytic index = (Total number of CD11b$^+$ cells taken up into macrophages/Total number of macrophages counted) $\times$ (Number of macrophages containing CD11b$^+$ cells taken up/Total number of macrophages counted) $\times$ 100

<Results>

The results are shown in Fig. 17. Hu NO. 1U (Humanized NO. 1) significantly enhanced phagocytosis (efferocytosis) of apoptotic thymocytes by macrophages.

It was revealed that Hu NO. 1U promotes phagocytosis induced by the binding of human CD300A to apoptotic cells and functionally inhibits (neutralizes) human CD300A.

[Effect of anti-human CD300A antibody on spinal cord injury]

<Method>

An anti-CD300a antibody was administered immediately after injury in a mouse spinal cord contusion injury model, and the effects on motor function recovery and secondary injury reduction were compared with a control group to evaluate the therapeutic efficacy of the anti-CD300a antibody for spinal cord injury.

Twenty female C57BL/6 mice (8 to 10 weeks old, about 25 g) were divided into two groups of ten mice each, which were designated as an antibody administration group and a control group.

The spinal cord contusion injury model was created by performing a laminectomy at the ninth thoracic vertebra under general anesthesia and using an Infinite Horizon impactor (IH impactor: manufactured by Precision Instruments, USA). The injury severity was set to 70 kdyn to induce a moderate spinal cord injury that allows partial recovery of hind limb function in mice. The spinal cord contusion injury model using the IH impactor is globally recognized as a standard in the field of spinal cord injury research.

During the acute phase after spinal cord injury, mice develop urinary retention, and thus, manual bladder expression was performed. Acetaminophen syrup was orally administered as an oral analgesic, and cefazolin as an antibiotic dissolved in physiological saline was administered by subcutaneous injection once daily for 3 days starting immediately after surgery to prevent, for example, urinary tract infections.

In this mouse spinal cord contusion injury model, the antibody administration group received an intraperitoneal injection of 400 μg of anti-CD300a antibody (EX42) immediately after injury, while the control group received an intraperitoneal injection of 400 μg of isotype control antibody. EX42 is an anti-mouse CD300a monoclonal antibody that has the function of inhibiting (neutralizing) the binding between CD300a and PS (Y. Wang, C. Nakahashi-Oda, Y. Okayama, A. Shibuya, Autonomous regulation of IgE-mediated mast cell degranulation and immediate hypersensitivity reaction byan inhibitory receptor CD300a. J. Allergy Clin. Immunol. 144, 323-327. e7 (2019)).

On the second day after spinal cord injury, mice that were able to walk using their hind limbs were excluded, and only mice with paralysis of both hind limbs were used for subsequent tests.

(Behavioral evaluation)

After spinal cord injury, behavioral evaluation was performed over time using the Basso Mouse Scale. The degree of hind limb functional recovery in each group was observed up to 6 weeks after injury.

The Basso Mouse Scale is as described in D. MICHELE BASSO et al., Basso Mouse Scale for Locomotion Detects Differences in Recovery after Spinal Cord Injury in Five Common Mouse Strains, JOURNAL OF NEUROTRAUMA, Volume 23, Number 5, 2006.

Specifically, behavior was observed for 4 minutes, and the degree of paralysis (walking impairment) was evaluated on a 10-point scale from 0 to 9, which was recorded as the BMS score. The BMS score is such that the lower the value, the more severe the paralysis, and 3 is the boundary for whether walking is possible. Furthermore, detailed observations not reflected in the BMS score were evaluated separately for the left and right hind limbs on an 11-point scale and recorded as the BMS subscore. As statistical analysis, two-way repeated measurement analysis of variance was performed, and then the Bonferroni's test was performed.

(Histological evaluation)

In the mouse spinal cord injury model, cavity and scar formation become apparent around 6 weeks after injury, and the condition is considered histologically to be chronic spinal cord injury, and therefore, behavioral evaluation was performed up to 6 weeks after injury. After completion of behavioral evaluation, the mice were euthanized, and the spinal cords were excised for analysis by histological evaluation.

The excised spinal cords were fixed in formalin and then embedded in paraffin. Horizontal sections were prepared at the center of the injured site (Epi-center), 1 mm rostral to the center of the injured site (Rostral + 1 mm), and 1 mm caudal to the center of the injured site (Caudal + 1 mm).

The prepared sections were stained with hematoxylin eosin (HE) by using a conventional method and observed with an optical microscope. In the spinal cord contusion injury model, the injured site and the non-injured site in HE-stained horizontal sections of the spinal cord typically have a distinct boundary, making it possible to measure the area of the injured site. Using this method, the area of the spinal cord injury was measured, and Spinal cord injury (SCI) area/Total area was calculated. As statistical analysis, unpaired multiple two-tailed Student's t-tests were performed.

Another section at the center of the injury site was stained with LUXOL Fast Blue (LFB) by a conventional method and observed with an optical microscope. In the LFB staining, only normal myelin in the white matter is intensely stained blue, and therefore, in the spinal cord contusion injury model, it is possible to evaluate the area of residual undamaged myelin by measuring the area of the intensely blue-stained portion. Using this method, the area of the portion stained with LFB in the central part of the spinal cord injury was measured, and LFB positive area/Total area was calculated. As statistical analysis, the Man-Whitney U test was performed.

<Results>

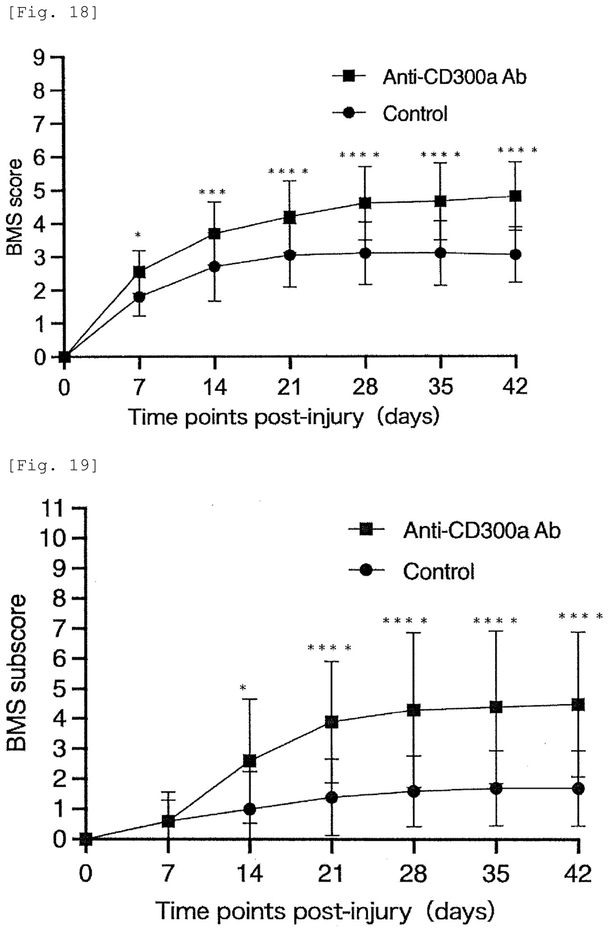

The results of the behavioral evaluation are shown in Figs. 18 and 19. The vertical axis in Fig. 18 indicates the BMS score, the vertical axis in Fig. 19 indicates the BMS subscore, and the horizontal axes in both drawings indicate the number of elapsed days after injury. The error bars indicate $\pm$ standard deviation, * indicates $p < 0.05$, *** indicates $p < 0.001$, and **** indicates $p < 0.0001$.

From Figs. 18 and 19, it was revealed that the scores of the antibody administration group were significantly higher than those of the control group, indicating that administration of the anti-CD300a antibody resulted in a significant improvement in behavioral evaluation. In particular, from Fig. 18, the BMS score remained at or below 3 throughout the observation period in the control group, whereas in the antibody administration group, the BMS score exceeded 3 after 14 days post-injury. That is, the control group was unable to walk throughout the observation period, whereas the antibody administration group was able to walk after 14 days post-injury.

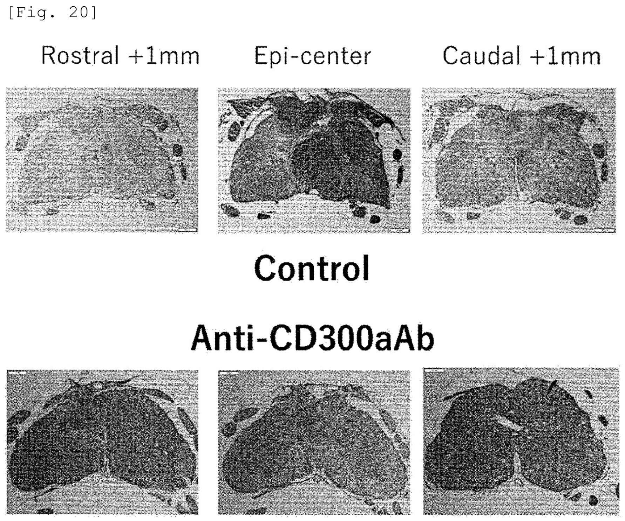

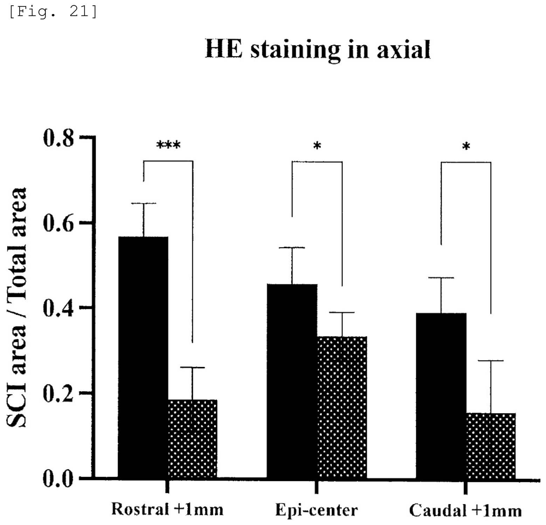

The results of HE staining are shown in Fig. 20, and the results of SCI area/Total area are shown in Fig. 21. In Fig. 21, the left column (black-filled) represents the control group, and the right column represents the antibody administration group. In Fig. 21, the error bars indicate $\pm$ standard deviation, * indicates $p < 0.05$, and *** indicates $p < 0.001$.

From Figs. 20 and 21, it was revealed that the SCI area/Total area in the antibody administration group was significantly lower than that in the control group, indicating that administration of the anti-CD300a antibody significantly reduced the area of spinal cord injury.

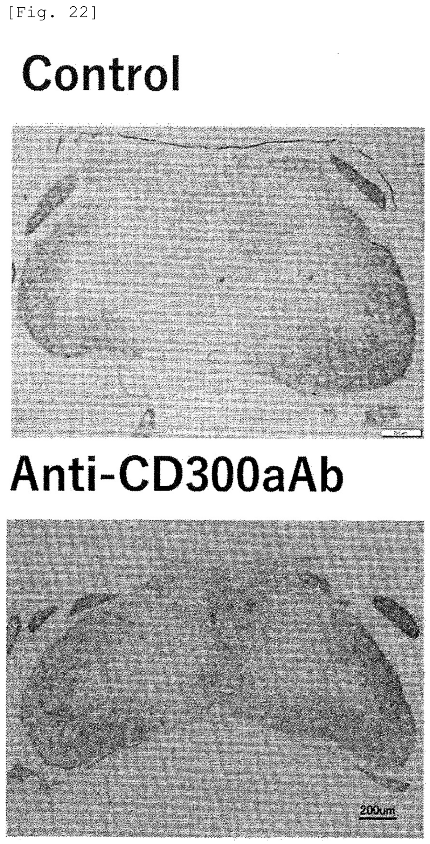

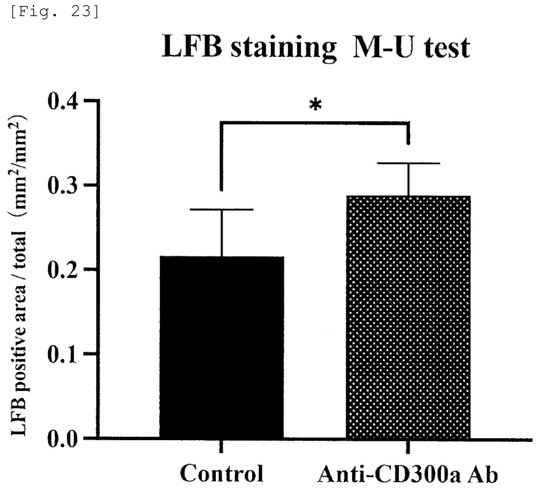

The results of LFB staining are shown in Fig. 22, and the results of LFB positive area/Total area are shown in Fig. 23. In Fig. 23, the error bars indicate $\pm$ standard deviation, and * indicates $p < 0.05$.

From Figs. 22 and 23, it was revealed that the LFB positive area/Total area in the antibody administration group was significantly higher than that in the control group, indicating that administration of the anti-CD300a antibody significantly increased the area of remaining myelin.

These results showed that, in the mouse spinal cord injury model, the anti-CD300a antibody alleviated neurological damage and promoted functional recovery. In addition, it was suggested that in humans as well, the anti-CD300a antibody can treat spinal cord injury, particularly by preventing and/or treating secondary injury.

It is known that the pathology of spinal cord injury includes primary injury and secondary injury. The primary injury is a mechanical damage caused by external force, and the degree thereof is defined by the mechanism of injury and/or the magnitude of the external force. On the other hand, the secondary injury refers to a process in which, after the primary injury, a biological reaction such as cell death of neuronal cells and glial cells associated with an increase in inflammatory cytokines, demyelination associated with cell death of oligodendrocytes, and disruption of the blood spinal cord barrier associated with disruption of the microvascular network is initiated, and tissue damage expands. In the damaged spinal cord tissue, the migration of myeloid cells such as neutrophils and macrophages is enhanced, and macrophages phagocytose dead cells.

Considering reports that inhibition of the interaction between PS and CD300a using an anti-CD300a antibody suppresses CD300a activity and promotes CD300b-dependent phagocytosis by macrophages, it is presumed that the anti-CD300a antibody promotes the phagocytosis of dead cells by macrophages generated during secondary spinal cord injury, thereby alleviating neural damage and contributing to functional recovery.

These results revealed that spinal cord injury is a disease that is caused by or associated with the function of human CD300A in suppressing the phagocytosis of dead cells by macrophages.

SEQ ID NO: 1: Amino acid sequence of HCDR1 of Antibody NO. 1
SEQ ID NO: 2: Amino acid sequence of HCDR2 of Antibody NO. 1
SEQ ID NO: 3: Amino acid sequence of HCDR3 of Antibody NO. 1
SEQ ID NO: 4: Amino acid sequence of LCDR1 of Antibody NO. 1
SEQ ID NO: 5: Amino acid sequence of LCDR2 of Antibody NO. 1
SEQ ID NO: 6: Amino acid sequence of LCDR3 of Antibody NO. 1
SEQ ID NO: 7: Amino acid sequence of heavy chain variable region of Antibody NO. 1
SEQ ID NO: 8: Amino acid sequence of light chain variable region of Antibody NO. 1
SEQ ID NO: 9: Amino acid sequence of HCDR1 of antibody NO. 2
SEQ ID NO: 10: Amino acid sequence of HCDR2 of antibody NO. 2
SEQ ID NO: 11: Amino acid sequence of HCDR3 of antibody NO. 2
SEQ ID NO: 12: Amino acid sequence of LCDR1 of antibody NO. 2
SEQ ID NO: 13: Amino acid sequence of LCDR2 of antibody NO. 2
SEQ ID NO: 14: Amino acid sequence of LCDR3 of antibody NO. 2
SEQ ID NO: 15: Amino acid sequence of heavy chain variable region of antibody NO. 2
SEQ ID NO: 16: Amino acid sequence of light chain variable region of antibody NO. 2
SEQ ID NO: 17: Amino acid sequence of HCDR1 of antibody NO. 3
SEQ ID NO: 18: Amino acid sequence of HCDR2 of antibody NO. 3
SEQ ID NO: 19: Amino acid sequence of HCDR3 of antibody NO. 3
SEQ ID NO: 20: Amino acid sequence of LCDR1 of antibody NO. 3
SEQ ID NO: 21: Amino acid sequence of LCDR2 of antibody NO. 3
SEQ ID NO: 22: Amino acid sequence of LCDR3 of antibody NO. 3
SEQ ID NO: 23: Amino acid sequence of heavy chain variable region of antibody NO. 3
SEQ ID NO: 24: Amino acid sequence of light chain variable region of antibody NO. 3
SEQ ID NO: 25: Amino acid sequence of HCDR1 of Hu NO. 1U
SEQ ID NO: 26: Amino acid sequence of HCDR2 of Hu NO. 1U
SEQ ID NO: 27: Amino acid sequence of HCDR3 of Hu NO. 1U
SEQ ID NO: 28: Amino acid sequence of LCDR1 of Hu NO. 1U
SEQ ID NO: 29: Amino acid sequence of LCDR2 of Hu NO. 1U
SEQ ID NO: 30: Amino acid sequence of LCDR3 of Hu NO. 1U
SEQ ID NO: 31: Amino acid sequence of heavy chain variable region of Hu NO. 1U
SEQ ID NO: 32: Amino acid sequence of light chain variable region of Hu NO. 1U
SEQ ID NO: 33: Base sequence of heavy chain variable region of Hu NO. 1U
SEQ ID NO: 34: Base sequence of light chain variable region of Hu NO. 1U
SEQ ID NO: 35: Amino acid sequence of heavy chain of Hu NO. 1U
SEQ ID NO: 36: Amino acid sequence of light chain of Hu NO. 1U
SEQ ID NO: 37: Base sequence of heavy chain of Hu NO. 1U
SEQ ID NO: 38: Base sequence of light chain of Hu NO. 1U
SEQ ID NO: 39: Partial amino acid sequence of human CD300A-R
SEQ ID NO: 40: Partial amino acid sequence of human CD300A-Q
SEQ ID NO: 41: Partial amino acid sequence of human CD300C
SEQ ID NO: 42: Partial amino acid sequence of Macaca fascicularis CD300A

## Claims

1. An anti-human CD300A monoclonal antibody or an antigen-binding fragment thereof that satisfies the following requirement (i), (ii), or (iii):

   (i) the anti-human CD300A monoclonal antibody or the antigen-binding fragment thereof including CDR1, CDR2 and CDR3 sequences of a heavy chain variable region having the amino acid sequence of SEQ ID NO: 7, and CDR1, CDR2 and CDR3 sequences of a light chain variable region having the amino acid sequence of SEQ ID NO: 8;

(ii) the anti-human CD300A monoclonal antibody or the antigen-binding fragment thereof including CDR1, CDR2 and CDR3 sequences of a heavy chain variable region having the amino acid sequence of SEQ ID NO: 15, and CDR1, CDR2 and CDR3 sequences of a light chain variable region having the amino acid sequence of SEQ ID NO: 16; or

(iii) the anti-human CD300A monoclonal antibody or the antigen-binding fragment thereof including CDR1, CDR2 and CDR3 sequences of a heavy chain variable region having the amino acid sequence of SEQ ID NO: 23, and CDR1, CDR2 and CDR3 sequences of a light chain variable region having the amino acid sequence of SEQ ID NO: 24.

2. The anti-human CD300A monoclonal antibody or the antigen-binding fragment thereof according to claim 1, wherein

the requirement (i) is satisfied,
the CDR1, CDR2, and CDR3 sequences of the heavy chain variable region are the amino acid sequences of SEQ ID NOs: 1, 2, and 3, respectively, and
the CDR1, CDR2, and CDR3 sequences of the light chain variable region are the amino acid sequences of SEQ ID NOs: 4, 5, and 6, respectively.

3. The anti-human CD300A monoclonal antibody or the antigen-binding fragment thereof according to claim 2, wherein the anti-human CD300A monoclonal antibody or the antigen-binding fragment thereof is a humanized antibody.

4. The anti-human CD300A monoclonal antibody or the antigen-binding fragment thereof according to claim 3, wherein the anti-human CD300A monoclonal antibody or the antigen-binding fragment thereof includes:

a heavy chain variable region having an amino acid sequence that is at least 95% identical to the amino acid sequence of SEQ ID NO: 31; and
a light chain variable region having an amino acid sequence that is at least 95% identical to the amino acid sequence of SEQ ID NO: 32.

5. The anti-human CD300A monoclonal antibody or the antigen-binding fragment thereof according to claim 2, wherein the anti-human CD300A monoclonal antibody or the antigen-binding fragment thereof includes:

a heavy chain variable region having the amino acid sequence of SEQ ID NO: 31; and
a light chain variable region having the amino acid sequence of SEQ ID NO: 32.

6. The anti-human CD300A monoclonal antibody or the antigen-binding fragment thereof according to claim 1, wherein

the requirement (ii) is satisfied,
the CDR1, CDR2, and CDR3 sequences of the heavy chain variable region are the amino acid sequences of SEQ ID NOs: 9, 10, and 11, respectively, and
the CDR1, CDR2, and CDR3 sequences of the light chain variable region are the amino acid sequences of SEQ ID NOs: 12, 13, and 14, respectively.

7. The anti-human CD300A monoclonal antibody or the antigen-binding fragment thereof according to claim 6, wherein the anti-human CD300A monoclonal antibody or the antigen-binding fragment thereof includes:

a heavy chain variable region having an amino acid sequence that is at least 80% identical to the amino acid sequence of SEQ ID NO: 15; and
a light chain variable region having an amino acid sequence that is at least 80% identical to the amino acid sequence of SEQ ID NO: 16.

8. The anti-human CD300A monoclonal antibody or the antigen-binding fragment thereof according to claim 1, wherein

the requirement (iii) is satisfied,
the CDR1, CDR2, and CDR3 sequences of the heavy chain variable region are the amino acid sequences of SEQ ID NOs: 17, 18, and 19, respectively, and
the CDR1, CDR2, and CDR3 sequences of the light chain variable region are the amino acid sequences of SEQ ID NOs: 20, 21, and 22, respectively.

9. The anti-human CD300A monoclonal antibody or the antigen-binding fragment thereof according to claim 8, wherein the anti-human CD300A monoclonal antibody or the antigen-binding fragment thereof includes:

a heavy chain variable region having an amino acid sequence that is at least 80% identical to the amino acid sequence of SEQ ID NO: 23; and
a light chain variable region having an amino acid sequence that is at least 80% identical to the amino acid sequence of SEQ ID NO: 24.

10. A nucleic acid encoding the anti-human CD300A monoclonal antibody or the antigen-binding fragment thereof according to claim 1.

11. A vector comprising the nucleic acid according to claim 10.

12. A transformant comprising the vector according to claim 11.

13. A pharmaceutical composition for treating or preventing a disease caused by a function of human CD300A, the pharmaceutical composition comprising the anti-human CD300A monoclonal antibody or the antigen-binding fragment thereof according to claim 3.

14. The pharmaceutical composition according to claim 13, wherein the disease is at least one selected from the group consisting of ischemic diseases, inflammatory diseases, infectious diseases, allergic diseases, organ fibrosis, and autoimmune diseases.

15. The pharmaceutical composition according to claim 13, wherein the disease is spinal cord injury.

16. A pharmaceutical composition for treating or preventing a disease associated with a function of human CD300A, the pharmaceutical composition comprising the anti-human CD300A monoclonal antibody or the antigen-binding fragment thereof according to claim 3.

17. The pharmaceutical composition according to claim 16, wherein the disease is at least one selected from the group consisting of ischemic diseases, inflammatory diseases, infectious diseases, allergic diseases, organ fibrosis, autoimmune diseases, and spinal cord injury.

18. A diagnostic agent comprising the anti-human CD300A monoclonal antibody or the antigen-binding fragment thereof according to claim 6 or 8.

[Fig. 1]

AMINO ACID SEQUENCES OF Human CD300A-R,
CD300A-Q, AND CD300C

```
                                                          56
huCD300A-R   ----LSKCRTVAGPVGGSLSVQCPYEKEHRTLNKYWCRPPQIE LCDKIVETKGSAGKRNG
huCD300A-Q   ----LSKCRTVAGPVGGSLSVQCPYEKEHRTLNKYWCRPPQIE LCDKIVETKGSAGKRNG
huCD300C     GYFPLSHPMTVAGPVGGSLSVQCRYEKEHRTLNKFWCRPPQILRCDKIVETKGSAGKRNG
             **:  *************** ***********:*******:  ***************

                                                106    112  115
huCD300A-R   RVSIRDSPANLSFTVTLENLTEEDAGTYWCGV DTPWLRDFHD PVVEVEVSVFPASTSMTP
huCD300A-Q   RVSIRDSPANLSFTVTLENLTEEDAGTYWCGV DTPWLQDFHD PVVEVEVSVFPASTSMTP
huCD300C     RVSIRDSPANLSFTVTLENLTEEDAGTYWCGVDTPWLRDFHDPIVEVEVSVFPAGTTTAS
             ******************************** *****:*********:.*: :.

huCD300A-R   ASITAAKTSTITTAFPPVSSTTLFAVGATHSASIQEETEEVVNSQLP
huCD300A-Q   ASITAAKTSTITTAFPPVSSTTLFAVGATHSASIQEETEEVVNSQLP
huCD300C     SPQSSMGTSGPPTKLPVHTWPSVTRKDSPEPSPHPGSLFSNVR----
             :. :: **  .* :*  : .::   .:....:.  .  . *.
```

☐ PREDICTED PS-BINDING REGION

⌐ ¬
¦ ¦ R、Q
└ ┘

[Fig. 2]

CD300A-R  CD300A-Q  CD300C
BW        /BW        /BW        /BW

TX49

No.1

No.2

No.3

huCD300A ⟶

EP 4 703 387 A1

[Fig. 3]

34

[Fig. 4]

[Fig. 5]

[Fig. 6]

[Fig. 7]

AMINO ACID SEQUENCES OF Human CD300A-R AND MACACA
FASCICULARIS (M. f.) CD300A

```
huCD300A-R    -----LSKCRTVAGPVGGSLSVQCPYEKEHRTLNKYWCRPPQIF CDKIVETKGSAGKRN
M.f.CD300A    LRGFALRGPSTVDGPVGGSLSVQCRYEEKHKIFNKYWCRPPRVL RCAKIVETEGPAGKMN
               *     ** ********** **::*: :*********::: * *****:*.*** *

huCD300A-R    GRVSIRDSPANLSFTVTLENLTEEDAGTYWCGV DTPWLRDFHD PVVEVEVSVFPASTSMT
M.f.CD300A    GRVSIMDCPQNLSFTVTLENLTEEDAGTYWCGV DTPWHQDLYD PYAQVEVSVFPASTSMT
               **** *.* **************************   :*::** .:************

huCD300A-R    PASITAAKTSTITTAFPPVSSTTLFAVGATHSASIQEETEEVVNSQLP
M.f.CD300A    PTSITAAKTSTITTVFPPVSSTSLFAVSATHSTSNWKENEEVVSSQLP
               *:***********.******:****.****:*   :*.****.****
```

☐ PREDICTED PS-BINDING REGION
⬚ R、Q

[Fig. 8]

No.1        No.2

MONOCYTES

LYMPHOCYTES

huCD300A ⟶

☐ CONTROL          ■ ANTI-CD300A
   ANTIBODY             ANTIBODY

[Fig. 9]

VH

```
No.2 MCPMSSPQTLNTLTPTMGWSWIFLFLLSGTAGVHSEVQLQQSGPELVKPGASMKISCKAS
No.3 ----------------MAWVWTLLLLMAAAQSAQAQIQLVQSGPELKKPGETVKISCKAS
No.1 ----------------MNFGLSLIFLALILKGVQCEVQLVESGGDLVKPGGSLKLSCAAS


No.2 GYSFTGYTMNWVKQSHGKNLEWIGLINPYNGGTSYNQKFKGKATLTVDKSSSTAYMELLS
No.3 GYTFTHYGLNWVKQAPGKGLQWMGWIHTYTGESTYADDFKGRSAFSLETSASTAYLQINN
No.1 GFTFSSYGMSWVRQTPDKRLEWVATISSGGSYTYYPDSVKGRFTISRDNAKNTLYLQMSS


No.2 LTSEDSAVYYCASG-------AMDYWGQGTSVTVSS
No.3 LKNEDTATYFCARGG----KDALDYWGQGTSVTVSS
No.1 LKSEDTAMYYCARHGDYGYPWFAYWGQGTLVTVSA
```

VL

```
No.2 ---MKLPVRPLVLMFWIPASSSDAVMTQTPLSLPVSLGDQASISCRSSQSLENSNGNTYL
No.3 --MEKDTLLLWVLLLWVPGSKGDIVLTQSPASLAVSLGQRATISCRASESVDN-YGISFM
No.1 MDFQVQIFSFLLISASVIMSRGQIVLTQSPAIMSASLGERVTMTCTASSSVSS----SYL


No.2 NWYLQKPGQSPQLLIYRVSNRFSGVLDRFSGSGSGTDFTLKISRVEAEDLGVYFCLQVTH
No.3 NWFQQKPGQSPKLLIYAASNQGSGVPARFSGSGSGTDFSLNIHPMEEDDIAMYFCHQSKE
No.1 HWYQQKPGSSPKLWIYSTSNLASGVPARFSGSGSGTSYSLTISSMEAEDAATYYCHQYHR


No.2 VPWTFGGGTKLEIK
No.3 VPWTFGGGTKLEIK
No.1 S-HTFGGGTKLEIK
```

[Fig. 10]

```
                          1          2          3
            123456789 0123456789 0123456789 0123456789
NO.1 VH     EVQLVESGG DLVKPGGSLK LSCAASGFTF SSYGMSWVRQ
HuNO.1 VH1  EVQLVESGG GLVQPGGSLR LSCAASGFTF SSYGMSWVRQ
HuNO.1 VH2  EVQLVESGG GLVQPGGSLR LSCAASGFTF SSYGMSWVRQ
HuNO.1 VH3  EVQLVESGG GLVQPGGSLR LSCAASGFTF SSYGMSWVRQ
U96282 VH   EVQLVESGG GLVQPGGSLR LSCAASGFTF S-----WVRQ


            4          5          6          7
            0123456789 01223456789 0123456789 0123456789
                            a
NO.1 VH     TPDKRLEWVA TISSGGSYTYY PDSVKGRFTI SRDNAKNTLY
HuNO.1 VH1  APGKGLEWVA TISSGGSYTYY PDSVKGRFTI SRDNAKNSLY
HuNO.1 VH2  APGKRLEWVA TISSGGSYTYY PDSVKGRFTI SRDNAKNSLY
HuNO.1 VH3  APDKRLEWVA TISSGGSYTYY PDSVKGRFTI SRDNAKNSLY
U96282 VH   APGKGLEWVA ----------- ------RFTI SRDNAKNSLY



                                       1          1
            8           9              0          1
            0122223456789 0123456789 0000123456789 0123
               abc                      abc
NO.1 VH     LQMSSLKSEDTAM YYCARHGDYG YPWFAYWGQGTLV TVSA
HuNO.1 VH1  LQMNSLRAEDTAV YYCARHGDYG YPWFAYWGQGTMV TVSS
HuNO.1 VH2  LQMNSLRAEDTAV YYCARHGDYG YPWFAYWGQGTMV TVSS
HuNO.1 VH3  LQMNSLRAEDTAV YYCARHGDYG YPWFAYWGQGTMV TVSS
U96282 VH   LQMNSLRAEDTAV YYCAR----- ------WGQGTMV TVSS
```

[Fig. 11]

```
                          1          2           3
            123456789 0123456789 01234567789 0123456789
                                      a
NO.1 VL     QIVLTQSPA IMSASLGERV TMTCTASSSVS SSYLHWYQQK
HuNO.1 VL1  DIQMTQSPS SLSASVGDRV TITCTASSSVS SSYLHWYQQK
HuNO.1 VL2  DIQMTQSPS SLSASVGDRV TITCTASSSVS SSYLHWYQQK
HuNO.1 VL3  QIQMTQSPS SLSASVGDRV TMTCTASSSVS SSYLHWYQQK
U96396 VL   DIQMTQSPS SLSASVGDRV TITC------- -----WYQQK


            4          5          6          7
            0123456789 0123456789 0123456789 0123456789
NO.1 VL     PGSSPKLWIY STSNLASGVP ARFSGSGSGT SYSLTISSME
HuNO.1 VL1  PGKAPKLWIY STSNLASGVP SRFSGSGSGT DYTLTISSLQ
HuNO.1 VL2  PGKAPKLWIY STSNLASGVP SRFSGSGSGT DFTLTISSLQ
HuNO.1 VL3  PGKAPKLWIY STSNLASGVP SRFSGSGSGT DYTLTISSLQ
U96396 VL   PGKAPKLLIY -------GVP SRFSGSGSGT DFTLTISSLQ


                                   1
            8          9           0
            0123456789 0123456789 01234567
NO.1 VL     AEDAATYYCH QY-HRSHTFG GGTKLEIK
HuNO.1 VL1  PEDFATYYCH QY-HRSHTFG GGTKVEIK
HuNO.1 VL2  PEDFATYYCH QY-HRSHTFG GGTKVEIK
HuNO.1 VL3  PEDFATYYCH QY-HRSHTFG GGTKVEIK
U96396 VL   PEDFATYYC- --------FG GGTKVEIK
```

[Fig. 12]

Heavy chain
AMINO ACID SEQUENCE

MNFGLSLIFLALILKGVQCEVQLVESGGGLVQPGGSLRLSCAASGFTFSSYGMSWVRQAPDKRLEWVATISSGGS
YTYYPDSVKGRFTISRDNAKNSLYLQMNSLRAEDTAVYYCARHGDYGYPWFAYWGQGTMVTVSSASTKGPSVFPL
APSSKSTSGGTAALGCLVKDYFPEPVTVSWNSGALTSGVHTFPAVLQSSGLYSLSSVVTVPSSSLGTQTYICNVN
HKPSNTKVDKKVEPKSCDKTHTCPPCPAPEAAGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTISKAKGQPREPQVYTLPP
SRDELTKNQVSLTCLVKGFYPSDIAVEWESNGQPENNYKTTPPVLDSDGSFFLYSKLTVDKSRWQQGNVFSCSVM
HEALHNHYTQKSLSLSPGK·

**bold:** variable region
white text: signal peptide
<u>underline</u>: CDRs


cDNA SEQUENCE


ATGAACTTCGGGCTCAGCTTGATTTTCCTTGCCCTCATTCTGAAAGGCGTCCAGTGTGAA
GTGCAGCTTGTGGAGTCTGGGGGAGGCCTTGTGCAGCCTGGAGGGTCCCTGAGACTCTCC
TGTGCAGCCTCTGGATTCACTTTCAGTAGCTATGGCATGTCTTGGGTTCGCCAGGCTCCA
GACAAGAGGCTGGAGTGGGTCGCAACCATTAGTAGTGGAGGCAGTTACACCTACTATCCA
GACAGTGTGAAGGGACGATTCACCATCTCCAGAGACAATGCCAAGAACAGCCTGTACCTG
CAAATGAACAGTCTGAGAGCTGAGGACACAGCCGTGTATTACTGTGCAAGACATGGGGAC
TATGGTTACCCCTGGTTTGCTTACTGGGGCCAAGGGACTATGGTCACTGTCTCTTCAGCC
TCCACCAAGGGCCCATCGGTCTTCCCCCTGGCACCCTCCTCCAAGAGCACCTCTGGGGGC
ACAGCGGCCCTGGGCTGCCTGGTCAAGGACTACTTCCCCGAACCGGTGACGGTGTCGTGG
AACTCAGGCGCCCTGACCAGCGGCGTGCACACCTTCCCGGCTGTCCTACAGTCCTCAGGA
CTCTACTCCCTCAGCAGCGTGGTGACCGTGCCCTCCAGCAGCTTGGGCACCCAGACCTAC
ATCTGCAACGTGAATCACAAGCCCAGCAACACCAAGGTGGACAAGAAAGTTGAGCCCAAA
TCTTGTGACAAAACTCACACATGCCCACCGTGCCCAGCACCTGAAGCTGCTGGAGGACCG
TCAGTCTTCCTCTTCCCCCCAAAACCCAAGGACACCCTCATGATCTCCCGGACCCCTGAG
GTCACATGCGTGGTGGTGGACGTGAGCCACGAAGACCCTGAGGTCAAGTTCAACTGGTAC
GTGGACGGCGTGGAGGTGCATAATGCCAAGACAAAGCCGCGGGAGGAGCAGTACAACAGC
ACGTACCGTGTGGTCAGCGTCCTCACCGTCCTGCACCAGGACTGGCTGAATGGCAAGGAG
TACAAGTGCAAGGTCTCCAACAAAGCCCTCCCAGCCCCCATCGAGAAAACCATCTCCAAA
GCCAAAGGGCAGCCCCGAGAACCACAGGTGTACACCCTGCCCCCATCCCGGGATGAGCTG
ACCAAGAACCAGGTCAGCCTGACCTGCCTGGTCAAAGGCTTCTATCCCAGCGACATCGCC
GTGGAGTGGGAGAGCAATGGGCAGCCGGAGAACAACTACAAGACCACGCCTCCCGTGCTG
GACTCCGACGGCTCCTTCTTCCTCTACAGCAAGCTCACCGTGGACAAGAGCAGGTGGCAG
CAGGGGAACGTCTTCTCATGCTCCGTGATGCATGAGGCTCTGCACAACCACTACACGCAG
AAGAGCCTCTCCCTGTCTCCGGGTAAATGA

[Fig. 13]

Light chain
AMINO ACID SEQUENCE

MDFQVQIFSFLLISASVIMSRGQIQMTQSPSSLSASVGDRVTMTCTASSSVSSSYLHWYQQKPGKAPKLWIYSTS
NLASGVPSRFSGSGSGTDYTLTISSLQPEDFATYYCHQYHRSHTFGGGTKVEIKRTVAAPSVFIFPPSDEQLKSG
TASVVCLLNNFYPREAKVQWKVDNALQSGNSQESVTEQDSKDSTYSLSSTLTLSKADYEKHKVYACEVTHQGLSS
PVTKSFNRGEC•

**bold**: variable region
white text: signal peptide
underline: CDRs

cDNA SEQUENCE

ATGGATTTTCAGGTGCAGATTTTCAGCTTCCTGCTCATCAGTGCCTCAGTCATCATGTCC
AGAGGACAGATTCAGATGACCCAGTCTCCATCATCCCTGTCTGCATCTGTTGGGGACCGG
GTCACCATGACCTGCACTGCCAGCTCAAGTGTTAGTTCCAGTTACTTGCACTGGTACCAG
CAGAAGCCAGGAAAAGCTCCCAAACTCTGGATCTATAGCACATCCAACCTGGCTTCTGGA
GTCCCATCTCGCTTCAGTGGCAGTGGGTCTGGGACCGATTACACTCTGACAATCAGCAGC
CTGCAGCCTGAAGATTTTGCCACTTATTACTGCCACCAGTATCATCGTTCCCACACATTC
GGAGGAGGGACCAAGGTGGAAATCAAACGAACTGTGGCTGCACCATCTGTCTTCATCTTC
CCGCCATCTGATGAGCAGTTGAAATCTGGAACTGCCTCTGTTGTGTGCCTGCTGAATAAC
TTCTATCCCAGAGAGGCCAAAGTACAGTGGAAGGTGGATAACGCCCTCCAATCGGGTAAC
TCCCAGGAGAGTGTCACAGAGCAGGACAGCAAGGACAGCACCTACAGCCTCAGCAGCACC
CTGACGCTGAGCAAAGCAGACTACGAGAAACACAAAGTCTACGCCTGCGAAGTCACCCAT
CAGGGCCTGAGCTCGCCCGTCACAAAGAGCTTCAACAGGGGAGAGTGTTAG

[Fig. 14]

huCD300A

☐ CONTROL      ■ HUMANIZED No. 1

[Fig. 15]

CD300A-R/BW       CD300A-Q/BW     ○ No.1    ▲ HUMANIZED No. 1

CONCENTRATION (ng/ml)     CONCENTRATION (ng/ml)

[Fig. 16]

CD300A-R Fc       CD300A-Q Fc

CONTROL   No.1   HUMANIZED No. 1     CONTROL   No.1   HUMANIZED No. 1

[Fig. 17]

$P = 0.0340$

CONTROL   HUMANIZED No. 1

[Fig. 18]

[Fig. 19]

[Fig. 20]

Rostral +1mm          Epi-center          Caudal +1mm

Control

Anti-CD300aAb

[Fig. 21]

HE staining in axial

[Fig. 22]

# Control

# Anti-CD300aAb

200um

[Fig. 23]

LFB staining  M-U test

## INTERNATIONAL SEARCH REPORT

| International application No. |
|---|
| **PCT/JP2024/012013** |

**A. CLASSIFICATION OF SUBJECT MATTER**

*C07K 16/28*(2006.01)i; *A61K 39/395*(2006.01)i; *A61P 1/04*(2006.01)i; *A61P 9/10*(2006.01)i; *A61P 29/00*(2006.01)i; *A61P 31/00*(2006.01)i; *A61P 37/02*(2006.01)i; *A61P 37/08*(2006.01)i; *A61P 43/00*(2006.01)i; *C12N 1/15*(2006.01)i; *C12N 1/19*(2006.01)i; *C12N 1/21*(2006.01)i; *C12N 5/10*(2006.01)i; *C12N 15/13*(2006.01)i; *C12N 15/63*(2006.01)i; *C12P 21/08*(2006.01)i; *G01N 33/53*(2006.01)i; *G01N 33/531*(2006.01)i

FI: C07K16/28; A61K39/395 N; A61P1/04; A61P9/10; A61P29/00; A61P31/00; A61P37/02; A61P37/08; A61P43/00 105; A61P43/00 107; A61P43/00 111; C12N1/15; C12N1/19; C12N1/21; C12N5/10; C12N15/13 ZNA; C12N15/63 Z; C12P21/08; G01N33/53 D; G01N33/53 V; G01N33/531 A

According to International Patent Classification (IPC) or to both national classification and IPC

**B. FIELDS SEARCHED**

Minimum documentation searched (classification system followed by classification symbols)

C07K1/00-19/00; C12N15/00-15/90; A61K39/395; A61P1/04; A61P9/10; A61P29/00; A61P31/00; A61P37/02; A61P37/08; A61P43/00; C12N1/15; C12N1/19; C12N1/21; C12N5/10; C12P21/08; G01N33/53; G01N33/531

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | LANKRY, D. et al. The interaction between CD300a and phosphatidylserine inhibits tumor cell killing by NK cells. Eur. J. Immunol. 2013, vol. 43, pp. 2151-2161 in particular, abstract, p. 2152, left column, 3rd paragraph to p. 2153, right column, 2nd paragraph, p. 2154, right column, 1st paragraph, p. 2159, left column, 3rd paragraph, fig. 1-2, 4 | 1-12, 18 |
| Y | in particular, abstract, p. 2152, left column, 3rd paragraph to p. 2153, right column, 2nd paragraph, p. 2154, right column, 1st paragraph, p. 2159, left column, 3rd paragraph, fig. 1-2, 4 | 13-17 |
| Y | WO 2014/073529 A1 (UNIVERSITY OF TSUKUBA) 15 May 2014 (2014-05-15) claims, paragraphs [0041]-[0053], [0277]-[0289], [0295] | 13-17 |

☑ Further documents are listed in the continuation of Box C.     ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **04 June 2024** | **18 June 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**

International application No.

**PCT/JP2024/012013**

| C. | DOCUMENTS CONSIDERED TO BE RELEVANT | |
|---|---|---|
| Category* | Citation of document, with indication, where appropriate, of the relevant passages | Relevant to claim No. |
| A | TAKAHASHI, M. et al. Human CD300C Delivers an Fc Receptor-γ-dependent Activating Signal in Mast Cells and Monocytes and Differs from CD300A in Ligand Recognition. J. Biol. Chem. 2013, vol. 288, no. 11, pp. 7662-7675<br>see entire text | 1-18 |
| P, X | KOIZUMI, H. et al. Development of Amonoclonal Antibodies Specific to Either CD300AR111 OR CD300AQ111 or Both. Monoclon. Antib. Immunodiagn. Immunother. 30 October 2023, vol. 42, no. 5, pp. 182-185<br>see entire text | 1-18 |

Form PCT/ISA/210 (second sheet) (July 2022)

**INTERNATIONAL SEARCH REPORT**

International application No.

**PCT/JP2024/012013**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

| | International application No. |
|---|---|
| | **PCT/JP2024/012013** |

| Patent document cited in search report | Publication date (day/month/year) | Patent family member(s) | Publication date (day/month/year) |
|---|---|---|---|
| WO 2014/073529 A1 | 15 May 2014 | US 2015/0299332 A1 claims, paragraphs [0133]-[0146], [0402]-[0416], [0429]-[0430] | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014073529 A

- WO 2019107445 A

### Non-patent literature cited in the description

- **BORREGO, F.** The CD300 molecules: an emerging family of regulators of the immunesystem.. *Blood*, 2013, vol. 121, 1951-1960
- **Y. OKOSHI et al.** Requirement of the tyrosines at residues 258 and 270 of MAIR-I in inhibitory effect on degranulation from basophilic leukemia RBL-2H3.. *Int. Immunol.*, 2005, vol. 17, 65-72
- **SIMHADRI, V.R. et al.** Human CD300A binds to phosphatidylethanolamine and phosphatidylserine, and modulates the phagocytosis of dead cells.. *Blood*, 2012, vol. 119, 2799-2809
- **VITALLAE, J. et al.** CD300A inhibits CD16-mediated NK cell effector functions in HIV-1-infected patients.. *Cell. Mol. Immnol.*, 2019, vol. 16, 940-942
- **ZENARRUZABEITIA, O. et al.** The expression and function of human CD300 receptors on blood circulating mononuclear cells are distinct in neonates and adults.. *Sci. Rep.*, 2016, vol. 6, 32693
- **BACHELET, I. et al.** The inhibitory receptor IRp60 (CD300A) is expressed and functional on human mast cells.. *J. Immunol.*, 2005, vol. 175, 7989-7995
- **YOTSUMOTO, K. et al.** Paired activating and inhibitory immunoglobulin-like receptors, MAIR-I and MAIR-II, regulate mast cell and macrophage activation.. *J. Exp. Med.*, 2003, vol. 198, 223-233
- **VITALLAE, J. et al.** Altered expression of CD300A inhibitory receptor on CD4+ T cells from human immunodeficiency virus-1-infected patients: association with disease progression markers.. *Front. Immunol.*, 2018, vol. 9, 1709
- **SIMHADRI, V.R. et al.** CD300C is an activating receptor expressed on human monocytes.. *J. Innate Immun.*, 2013, vol. 5, 389-400

- **TAKAHASHI M. et al.** Human CD300C delivers an Fc receptor-γ-dependent activating signal in mast cells and monocytes and differs from CD300A in ligand recognition.. *J. Biol. Chem.*, 2013, vol. 288, 7662-75
- Reference SNP (rs) Report rs2272111, dbSNP Short Genetic Variations, National Library of Medicine, [online. *National Center for Biotechnology Information*, 25 April 2023, <https://www.ncbi.nlm.nih.gov/snp/rs2272111>
- **SPECKMAN, R.A. et al.** Novel immunoglobulin superfamily gene cluster, mapping to a region of hman chromosome 17q25, linked to psoriasis ssceptibility.. *Hum. Genet.*, 2003, vol. 112, 31-41
- **KÖHLER** ; **MILSTEIN**. *Nature*, 1975, vol. 256, 495-497
- *Nature Biotechnology*, 2005, vol. 23, 1105
- **THOMAS TILLER**. *Journal of immunological methods*, 2009, vol. 350, 183-193
- **KABAT et al.** Sequences of Proteins of Immunological Interests. Department of Health and Human Services, 1991, vol. 91, 3242
- **KABAT et al.** Sequences of Proteins of Immunological Interests. Department of Health and Human Services, 1991, 91-3242
- *J. Allergy Clin. Immunol.*, 2019, vol. 144, 323-327, 7
- **D. MICHELE BASSO et al.** Basso Mouse Scale for Locomotion Detects Differences in Recovery after Spinal Cord Injury in Five Common Mouse Strains. *JOURNAL OF NEUROTRAUMA*, 2006, vol. 23 (5)